# EUROPEAN PATENT SPECIFICATION

(11) **EP 4 302 799 B1**
(45) Date of publication and mention of the grant of the patent: **27.08.2025**
(21) Application number: 22775074.2
(22) Date of filing: 08.03.2022
(51) Int. Cl.: A61M 5/142

(54) **PHARMACEUTICAL LIQUID ADMINISTRATION DEVICE**
VORRICHTUNG ZUR VERABREICHUNG EINER PHARMAZEUTISCHEN FLÜSSIGKEIT
DISPOSITIF D'ADMINISTRATION DE SOLUTION MÉDICAMENTEUSE

(30) Priority: 25.03.2021 JP 2021050938
(43) Date of publication of application: 10.01.2024
(73) Proprietor: TERUMO Kabushiki Kaisha, Tokyo 151-0072 (JP)
(72) Inventor: UCHIYAMA, Joji, Ashigarakami-gun, Kanagawa 259-0151 (JP); YAMAZAKI, Tsuyoshi, Ashigarakami-gun, Kanagawa 259-0151 (JP)
(74) Representative: TBK
(86) International application number: PCT/JP2022/009924
(87) International publication number: WO 2022/202280

(56) References cited:
- JP-A- 2011 507 555
- JP-A- 2015 500 101
- JP-A- 2016 523 123
- US-A1- 2007 123 819
- US-A1- 2011 213 306
- US-A1- 2015 250 943
- US-A1- 2019 133 505
- US-A1- 2019 351 134

## Description

### Technical Field

The present invention relates to a drug solution administration apparatus for administering a drug solution to a subject such as a living body.

### Background Art

A therapeutic method of continuously administering a drug solution into the body of a patient is known. For example, as a therapeutic method for a diabetic patient, a therapeutic method of continuously administering insulin in a trace amount to the patient is known. In the therapeutic method, a portable drug solution administration apparatus that can be carried by being fixed to a body or clothing of a user is used. In a case where the portable drug solution administration apparatus is used, a drug solution can be administered to the user all day. As such a type of drug solution administration apparatus, an insulin pump for administering insulin to a user is known.

As one portable drug solution administration apparatus described above, proposed is a drug solution administration apparatus that includes a syringe-like reservoir in which a drug solution is stored, and that includes a plunger to be driven inside the reservoir. In the drug solution administration apparatus, a cannula is connected in a liquid-tight manner to a drug solution supply pipe extending from the reservoir, the cannula is indwelled under the skin of a user, and the stored drug solution is administered into the body of the user.

PTL 1 describes a technique of providing a drug solution supply needle member (a drug solution supply needle) having a needle tube at a tip end portion of a drug solution supply pipe, and providing a rubber partition (a rubber plug) at a connection port having a cannula, and connecting the drug solution supply pipe and the cannula by puncturing the rubber plug using the drug solution supply needle.

### Citation List

### Patent Literature

PTL 1: European Patent No. EP 1 951 340 A1
PTL 2: US 2015/250943 A1
PTL 3: US 2019/351134 A1
PTL 4: US 2019/133505 A1
PTL 5: US 2011/213306 A1
PTL 6: US 2007/123819 A1

US 2015/250943 A1 discloses a liquid transport device which is attachable to a living body and transports a liquid into the living body. The liquid transport device includes a storage portion that stores the liquid; a pumping portion that transports the liquid in the storage portion to the living body by compressing a tube; and an injection portion that injects the liquid which is transported by the pump portion into the living body. The injection portion is disposed between the storage portion and the tube when viewed from the direction perpendicular to the surface of the living body to which the liquid transport device is attached. Such a liquid transport device is reduced in size.

US 2019/351134 A1 discloses a user-wearable patch pump system for delivery of insulin or other medicament. The system can include a pump and an attachment portion that attaches the pump to a user's body. The pump can include a drive unit and a disposable cartridge containing a medicament with the drive unit configured to cause the pump to deliver the medicament in the cartridge to the user. The attachment portion can include a retention frame configured to selectively retain the pump therein and an adhesive patch configured to be attached to the user's body. The pump can be selectively attached to the retention frame and used to deliver medicament either through a cannula to an infusion site directly beneath the retention frame or through tubing to an infusion site displaced from the retention frame.

US 2019/133505 A1 discloses a medical device for transcutaneously inserting an insertable element into a body tissue. The medical device includes an insertable element that has an in vivo distal end for subcutaneous insertion, an ex vivo proximal end, and a pre-bended insertion cannula for subcutaneously inserting the insertable element. The medical device further comprises at least one patch, which is configured to be mounted on the skin of a user. The patch has a patch base and an integrated insertion mechanism for driving the insertion cannula from a storage position within the patch into an inserted position within the body tissue on a curved insertion path.

US 2011/213306 A1 discloses a delivery system for delivering fluidic media to a user having a second housing portion configured to be selectively operatively engaged with and disengaged from a first housing portion, the first housing portion and the second housing portion configured to be slidable relative to each other to operatively engage each other; and a fluid connector supported by one the housing portions in a position to engage a reservoir supported by an other of the housing portions in a case where the first housing portion and the second housing portion are slid relative to each other to operatively engage each other.

US 2007/123819 A1 discloses an infusion pump system that increases patient comfort and convenience. The infusion pump system includes an infusion site interface that is releasably connected to an infusion pump body, and has no tubing associated between the infusion site interface and the pump body. The infusion pump body may include a carrier frame that may be adhered to the skin of a user.

### Summary of Invention

### Technical Problem

The technique described in PTL 1 has the following problems. A drug solution administration apparatus such as an insulin pump includes an apparatus body and a cradle that is attachable to and detachable from each other. The cradle is fixed by being affixed to the skin of a user, and the apparatus body is attached to and detached from the cradle. The syringe (the reservoir), the plunger, the drug solution supply pipe, and the drug solution supply needle described above are provided in the apparatus body, and the connection port described above is provided in the cradle. When mounting the apparatus body to the cradle, in a state where the rubber plug is punctured by the drug solution supply needle, the apparatus body is caused to rotationally move (swirl) with a punctured portion as a center, and is mounted to the cradle.

However, during use of the drug solution administration apparatus, it is necessary to detach the apparatus body from the cradle and mount the apparatus body to the cradle again for some reason, for example, when the user takes a bath or when the user performs filling of the drug solution. Therefore, attaching and detaching operations between the apparatus body and the cradle are repeated during the use of the drug solution administration apparatus, and puncturing and removing operations of the drug solution supply needle to the rubber plug are performed corresponding to the number of repetitions. As a result, for example, there is a risk that a punctured hole of the rubber plug is increased in size or the rubber plug is damaged, and the drug solution leaks from a connection portion between the rubber plug and the drug solution supply needle. In particular, in the apparatus disclosed in PTL 1, the apparatus body is caused to rotationally move and mounted to the cradle in a state of being punctured. At this time, since the drug solution supply needle is twisted in the rubber plug, the rubber plug is likely to be damaged.

Further, the apparatus disclosed in PTL 1 is often mounted to the abdomen or the back of the user, and when mounting the apparatus body to the cradle, a surface of the rubber plug may be punctured by the drug solution supply needle from an oblique direction. When the rubber plug is obliquely punctured by the drug solution supply needle, a punctured hole formed in the rubber plug is likely to be longer than that in a case where the surface of the rubber plug is punctured by the drug solution supply needle in a vertical direction, which may accelerate deterioration of the rubber plug.

At least one embodiment of the invention has been made in view of the circumstances described above, and specifically, an object of the invention is to provide a drug solution administration apparatus that is capable of preventing deterioration of members constituting a connection port during repetition of attaching and detaching operations between an apparatus body and a cradle, and improving mountability of the apparatus body to the cradle.

### Solution to Problem

The above object is solved by a drug solution administration apparatus having the features of claim 1. Further developments are subject matter of the dependent claims.

### Advantageous Effect of Invention

According to at least one embodiment of the invention, it is possible to prevent deterioration of members constituting a connection port during repetition of attaching and detaching operations between an apparatus body and a cradle, and to improve mountability of the apparatus body to the cradle.

### Brief Description of Drawings

[FIG. 1] FIG. 1 is a schematic perspective view of a drug solution administration apparatus according to a first embodiment.
[FIG. 2] FIG. 2 is a schematic exploded perspective view of the drug solution administration apparatus according to the first embodiment.
[FIG. 3] FIG. 3 is a schematic partial perspective view of the drug solution administration apparatus according to the first embodiment when seen from a bottom surface side.
[FIG. 4A] FIG. 4A is a schematic cross-sectional view when a rotating member is at a closed position in the drug solution administration apparatus according to the first embodiment.
[FIG. 4B] FIG. 4B is a schematic cross-sectional view when the rotating member is at an open position in the drug solution administration apparatus according to the first embodiment.
[FIG. 5] FIG. 5 is a schematic cross-sectional view illustrating operations of the drug solution administration apparatus according to the first embodiment.
[FIG. 6] FIG. 6 is a schematic cross-sectional view illustrating the operations of the drug solution administration apparatus according to the first embodiment.
[FIG. 7] FIG. 7 is a schematic cross-sectional view illustrating the operations of the drug solution administration apparatus according to the first embodiment.
[FIG. 8] FIG. 8 is a schematic cross-sectional view illustrating the operations of the drug solution administration apparatus according to the first embodiment.
[FIG. 9] FIG. 9 is a schematic perspective view of a cradle of a drug solution administration apparatus according to a second embodiment.
[FIG. 10] FIG. 10 is a schematic perspective view of the drug solution administration apparatus according to the second embodiment when seen from a bottom surface side.
[FIG. 11] FIG. 11 is a schematic cross-sectional view of a region close to a connection port of the drug solution administration apparatus according to the second embodiment.
[FIG. 12] FIG. 12 is a schematic cross-sectional view illustrating operations of the drug solution administration apparatus according to the second embodiment.
[FIG. 13] FIG. 13 is a schematic cross-sectional view illustrating the operations of the drug solution administration apparatus according to the second embodiment.

### Description of Embodiments

Hereinafter, embodiments for carrying out the invention will be described in detail with reference to the drawings. The embodiments described here are examples for embodying a technical idea of the invention, and do not limit the invention. Further, other aspects, examples, operational techniques, and the like that can be implemented by those skilled in the art without departing from the scope of the invention are all included in the scope of the invention, and are included in the invention described in the claims and the scope of equivalents thereof.

Further, in the drawings attached to the present description, for convenience of illustration and understanding, a scale, an aspect ratio, a shape, and the like may be changed from actual ones and may be schematically expressed as appropriate, and the drawings are just examples and do not limit the interpretation of the invention.

Further, in the following description, in a case where the description is given by adding an ordinal number such as "first" or "second", unless otherwise specified, it is used for convenience and does not define any order.

In the present description, for convenience of description, XYZ coordinates are set as illustrated. That is, a "Z direction" is a direction along a vertical direction, an "X direction" is a direction perpendicular to the Z direction and parallel to a horizontal plane, and a "Y direction" is another direction perpendicular to the Z direction and parallel to the horizontal plane (a direction perpendicular to the X direction). Therefore, in drug solution administration apparatuses 100, 110 according to the embodiments, the Z direction coincides with a thickness direction (an upward-downward direction) of the apparatuses, an upward direction is a direction from a living body surface toward the drug solution administration apparatuses 100, 110, and a downward direction is a direction toward the living body surface. The X direction coincides with a longitudinal direction (a front-rear direction) along an axial direction of the apparatuses, and the Y direction coincides with a transverse direction (a width direction) perpendicular to the longitudinal direction of the apparatuses.

The drug solution administration apparatuses 100, 110 according to the embodiments are each an apparatus for continuously administering a drug solution into a user. Examples of the drug solution to be administered using the drug solution administration apparatuses 100, 110 include insulin, analgesics, anti-cancer drugs, human immunodeficiency virus (HIV) drugs, iron chelators, and pulmonary hypertension drugs. In the embodiments, as an example of the drug solution administration apparatuses 100, 110, a portable insulin pump for administering insulin into the body of a user is assumed. The drug solution administration apparatuses 100, 110, which are insulin pumps, are used by being affixed to a surface of a living body. The surface of the living body is typically a surface of the skin of the user. Further, positions where the drug solution administration apparatuses 100, 110 are affixed are, for example, the abdomen of the user.

### [First Embodiment]

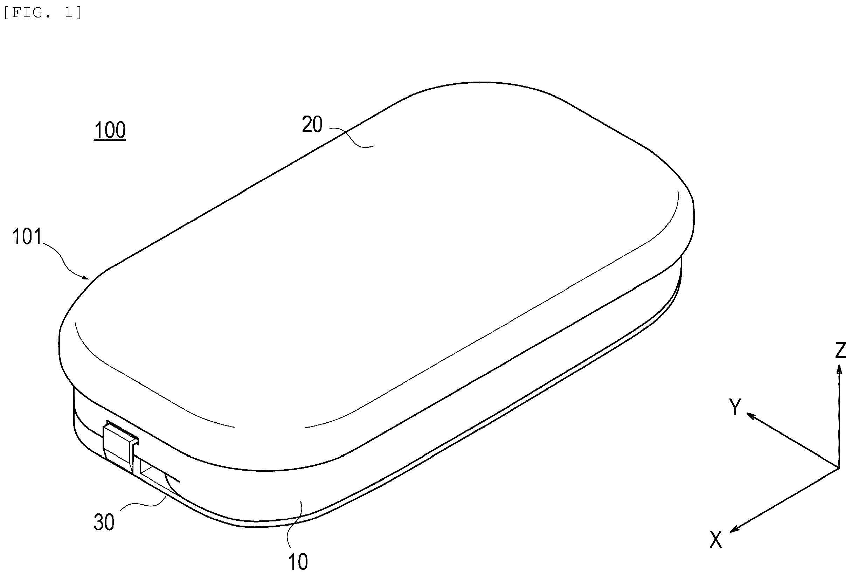

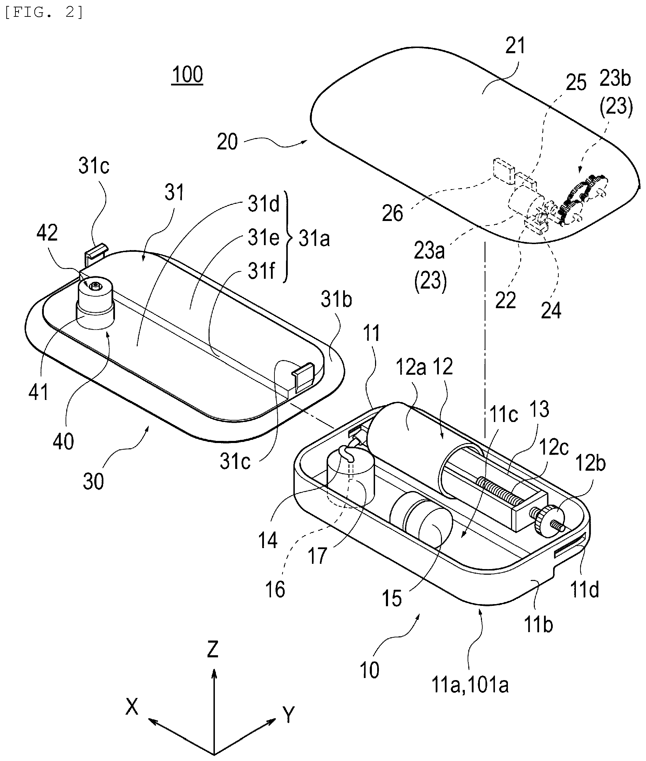

FIG. 1 is a schematic perspective view illustrating an appearance of the drug solution administration apparatus 100 according to a first embodiment, and FIG. 2 is a schematic exploded perspective view illustrating main parts of an overall configuration of the drug solution administration apparatus 100.

### <Configuration>

As illustrated in FIG. 1 or FIG. 2, in brief, the drug solution administration apparatus 100 includes a first body portion 10 that holds a reservoir 12 in which a drug solution is stored, and the like, a second body portion 20 that holds a drug solution supply driver 23 for supplying the drug solution in the reservoir 12 into a living body, and a cradle 30 that holds a cannula 50 and a connection port 40 and that is to be affixed to a living body surface.

The first body portion 10 is a disposable portion. The second body portion 20 is a reuse portion. The first body portion 10 and the second body portion 20 are separable from each other and are connectable to each other. In addition, the drug solution administration apparatus 100 is constituted by an apparatus body 101 in which the first body portion 10 and the second body portion 20 are connected to each other, and the cradle 30 to which the apparatus body 101 is mounted.

### <First Body Portion>

The first body portion 10 includes a housing 11, the reservoir 12, an extruding portion 13, a drug solution supply pipe 14, and a power source portion 15. The housing 11 is formed in a substantially rectangular shape in a plan view (seen in the Z direction). In addition, the housing 11 is a substantially rectangular parallelepiped with an upper opening.

As illustrated in FIG. 2, the housing 11 includes a flat plate-like bottom surface portion 11a and side wall portions 11b rising along the entire periphery of an outer peripheral edge of the bottom surface portion 11a. The bottom surface portion 11a is a plate portion that partitions the housing 11 in the Z direction. The bottom surface portion 11a is disposed to face the cradle 30 when the apparatus body 101 is mounted to the cradle 30. In the bottom surface portion 11a, the reservoir 12, the extruding portion 13, the drug solution supply pipe 14, and the power source portion 15 are mounted to a surface facing the second body portion 20 (an upper surface of the bottom surface portion 11a). The bottom surface portion 11a also functions as a bottom surface portion 101a of the apparatus body 101 in which the second body portion 20 is connected to the first body portion 10.

The housing 11 includes a first housing portion 11c surrounded by the bottom surface portion 11a and the side wall portions 11b. The first housing portion 11c is a space provided on an upper surface side of the bottom surface portion 11a, and houses the reservoir 12, the power source portion 15, and the like. When a lid 21 of the second body portion 20 and the housing 11 are connected to each other, the drug solution supply driver 23 attached to the inside of the lid 21 is housed in the first housing portion 11c.

The housing 11 includes first mounting portions 11d for engaging with second mounting portions 31c of the cradle 30, which will be described later, and maintaining a mounted state between the apparatus body 101 and the cradle 30 when the apparatus body 101 is mounted to the cradle 30. The first mounting portions 11d are each an engaging recess provided on an outer surface of the side wall portion 11b of the first body portion 10 along the Y direction. As illustrated in FIG. 2, the first mounting portions 11d are disposed at positions facing the second mounting portions 31c with respect to two corresponding side wall portions 11b (that is, the side wall portions 11b on short sides) along the Y direction in a state where the apparatus body 101 is mounted to the cradle 30.

The first mounting portions 11d and the second mounting portions 31c function as mounting portions for stably holding the mounted state between the apparatus body 101 and the cradle 30. Therefore, when the apparatus body 101 is mounted to the cradle 30, the drug solution administration apparatus 100 can stably maintain the mounted state.

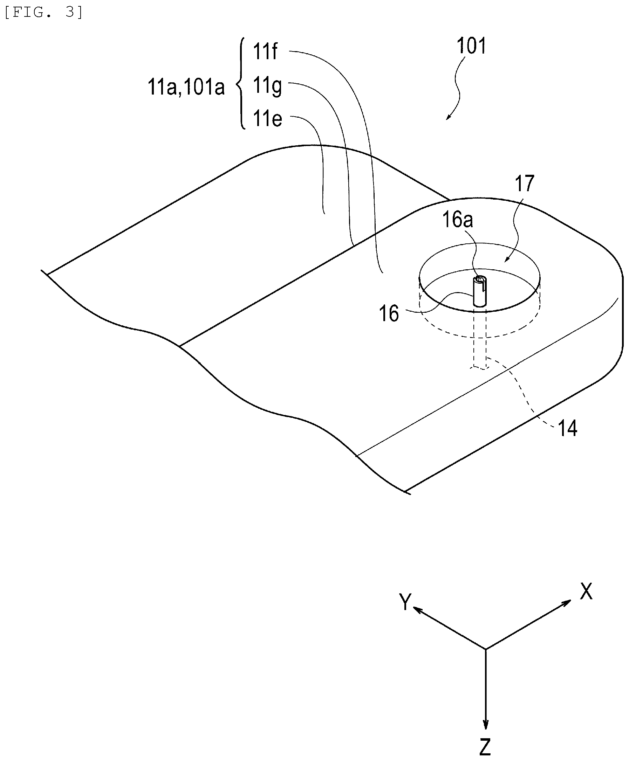

As illustrated in FIG. 3, the bottom surface portion 11a includes a first bottom surface 11e, a second bottom surface 11f, and a first step portion 11g. The first bottom surface 11e and the second bottom surface 11f are each a plate surface extending along the X direction. As an example, the first bottom surface 11e is disposed on a mounting direction D1 side of the apparatus body 101 in the bottom surface portion 11a, and as an example, the second bottom surface 11f is disposed on a mounted state release direction (a mounting release direction D2) side of the apparatus body 101 in the bottom surface portion 11a. The first bottom surface 11e and the second bottom surface 11f are positioned in a stepped manner in the thickness direction of the housing 11 (the Z direction), and the second bottom surface 11f is positioned below the first bottom surface 11e (on a living body surface side). The first bottom surface 11e is placed on a first placement surface 31e of a placement surface portion 31a when the apparatus body 101 is placed on the cradle 30. The second bottom surface 11f is placed on a second placement surface 31d of the placement surface portion 31a in a state where the apparatus body 101 is placed on the cradle 30.

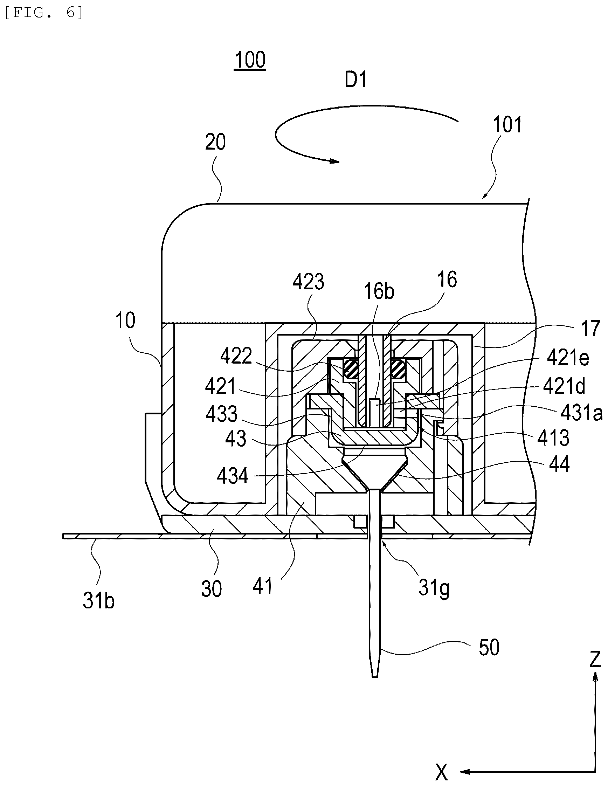

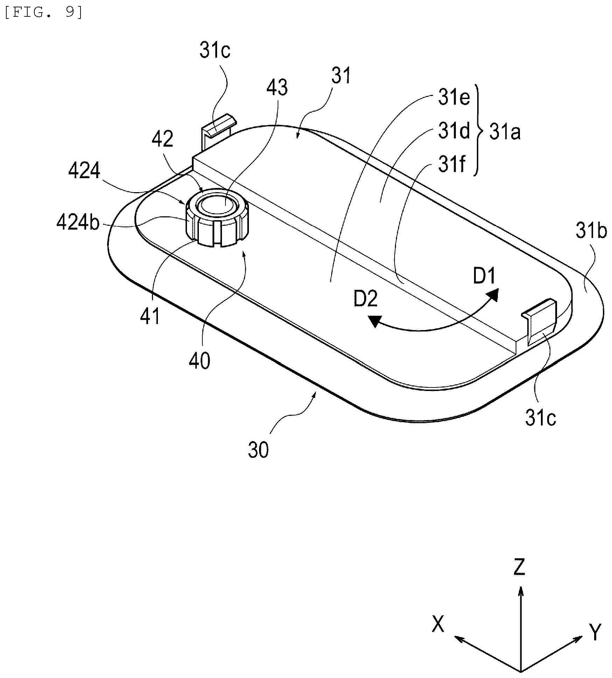

Here, the "mounting direction D1" is a direction that is substantially parallel to the placement surface portion 31a for causing the apparatus body 101 to rotationally move when mounting the apparatus body 101 to the cradle 30 (that is, substantially parallel to the living body surface) (see FIGS. 6 and 9). By causing the apparatus body 101 to rotate in the mounting direction D1 with an engaging position between an engaging recess 17 of the apparatus body 101 and the connection port 40 provided on the cradle 30 (specifically, a puncturing position of a drug solution supply portion 16 to a plug 43) as a rotation center, the bottom surface portion 101a (the bottom surface portion 11a) of the apparatus body 101 can be moved to above the placement surface portion 31a of the cradle 30.

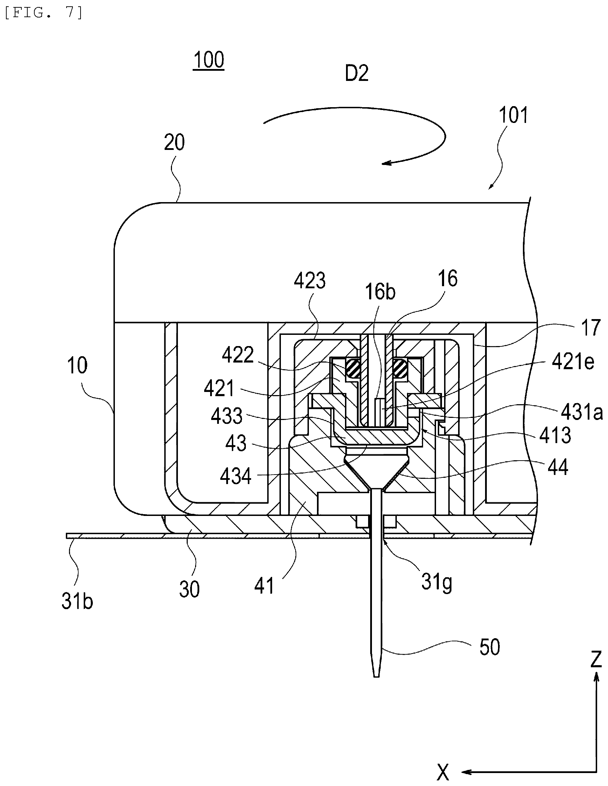

The "mounting release direction D2" is a direction that is opposite to the mounting direction D1 and is substantially parallel to the placement surface portion 31a for causing the apparatus body 101 to rotationally move when removing the apparatus body 101 from the cradle 30 (see FIG. 7). By causing the apparatus body 101 to rotate in the mounting release direction D2 with the engaging position between the engaging recess 17 of the apparatus body 101 and the connection port 40 provided on the cradle 30 as the rotation center, the bottom surface portion 101a of the apparatus body 101 can be moved to a position deviated from above the placement surface portion 31a of the cradle 30. When the apparatus body 101 rotationally moves in the mounting release direction D2 and is then lifted upward, the engagement between the engaging recess 17 and the connection port 40 is released, and the apparatus body 101 is completely detached from the cradle 30.

The first step portion 11g is provided in a boundary portion that defines the first bottom surface 11e and the second bottom surface 11f. The first step portion 11g extends to vertically cross the bottom surface portion 11a along the X direction. The first step portion 11g has a step surface that rises in a substantially vertical direction (the Z direction) from the second bottom surface 11f toward the first bottom surface 11e when the first body portion 10 is seen from a side surface (seen in the X direction). When mounting the apparatus body 101 to the cradle 30, the first step portion 11g is in contact with a step surface of a second step portion 31f of the cradle 30 to restrict the rotational movement of the apparatus body 101.

The bottom surface portion 11a may have a shape complementary to that of the placement surface portion 31a of the cradle 30 in a mounting completion state of the apparatus body 101 and the cradle 30. Therefore, a configuration of the bottom surface portion 11a is not limited to the configuration described above.

The reservoir 12 includes an outer cylinder 12a in which the drug solution to be administered to the user is stored, and discharges the drug solution in the outer cylinder 12a by a pressing operation by the extruding portion 13. The reservoir 12 is provided with a discharge port (not illustrated) for the drug solution at a tip end thereof, and one end of the drug solution supply pipe 14 is connected to the discharge port. The other end of the drug solution supply pipe 14 is connected to the drug solution supply portion 16 from a first housing portion 11c side of the housing 11. The reservoir 12 is provided with a gear 12b in a vicinity of an end portion thereof. The gear 12b rotates due to a driving force from the drug solution supply driver 23. A feed screw 12c meshes with an end portion of a rotation shaft of the gear 12b. The feed screw 12c is provided to be movable in the X direction. In the reservoir 12, a gear 23b and the gear 12b rotate by driving from the drug solution supply driver 23, and the extruding portion 13 moves in the X direction while the feed screw 12c rotates in accordance with the rotation of the gear 12b. The drug solution in the reservoir 12 is supplied to the drug solution supply pipe 14 depending on a pressing amount of the extruding portion 13 that moves by the rotation of the feed screw 12c. The drug solution supply pipe 14 is fluidly connected (in a connection state where the drug solution can flow) to the connection port 40, which will be described later, in a detachable manner via the drug solution supply portion 16.

The reservoir 12 is not limited to a syringe, and may be any reservoir capable of storing the drug solution, for example, a soft bag. The reservoir 12 is only required to be capable of discharging the drug solution by the extruding portion 13.

The power source portion 15 supplies a driving power supply necessary for driving the drug solution administration apparatus 100. The power source portion 15 includes a battery serving as a power supply for driving the drug solution supply driver 23 and the like, and a battery box for housing the battery. The power source portion 15 is connected to an electrode (not illustrated) on a circuit board 22. The power source portion 15 may be disposed inside the lid 21 as a component of the second body portion 20.

The drug solution supply portion 16 has a cylindrical hollow shape in which a drug solution supply hole 16a is formed at a tip end. A proximal end of the drug solution supply portion 16 is connected to the drug solution supply pipe 14, and the tip end of the drug solution supply portion 16 is mounted to the connection port 40 provided on the cradle 30. The drug solution supply hole 16a is formed to penetrate the drug solution supply portion 16 along a central axis of the drug solution supply portion 16. As illustrated in FIG. 3, the drug solution supply portion 16 is disposed in a manner of not protruding beyond the bottom surface portion 11a (the second bottom surface 11f) inside the engaging recess 17 provided in the bottom surface portion 11a of the first body portion 10.

A first engaging portion 16b to be engaged with a second engaging portion 421e of a rotating member 421, which will be described later, is provided at the tip end of the drug solution supply portion 16. The first engaging portion 16b is implemented by, for example, a notch groove that extends in an axial direction from the tip end toward the proximal end of the drug solution supply portion 16. When the apparatus body 101 is temporarily mounted to the cradle 30, the first engaging portion 16b is inserted into the connection port 40 and is engaged with the second engaging portion 421e. The first engaging portion 16b is engaged with the second engaging portion 421e when the drug solution supply portion 16 is mounted to the rotating member 421, and transmits a rotational force caused by the rotational movement of the apparatus body 101 in the mounting direction D1 to the rotating member 421 via the second engaging portion 421e.

The engaging recess 17 is provided to be recessed in the thickness direction of the housing 11 on the bottom surface portion 11a (specifically, the second bottom surface 11f) of the first body portion 10. An inner peripheral shape and an inner dimension of the engaging recess 17 are set depending on an outer shape and an outer dimension of the connection port 40 to be engaged therewith. The engaging recess 17 is engaged with the connection port 40 so as to cover an outer periphery of the connection port 40 when the apparatus body 101 is mounted to the cradle 30. The engaging recess 17 is disposed at a position in the first bottom surface 11e facing the connection port 40 when the apparatus body 101 is mounted to the cradle 30. The drug solution supply portion 16 is disposed at a substantially central portion of the engaging recess 17. For the purpose of further stabilizing a mounting operation, a structure such as a screw or a notch that is rotated and engaged relative to one another may be provided in an inner peripheral surface of the engaging recess 17 so as to engage with an outer peripheral surface of the connection port 40.

### <Second Body Portion>

The second body portion 20 includes the lid 21, the circuit board 22, and the drug solution supply driver 23, and is connected to the first body portion 10. The second body portion 20 is a portion where electronic control functions of the drug solution administration apparatus 100 are provided in an aggregated manner. The second body portion 20 is configured by housing electronic control functional components such as the circuit board 22 and the drug solution supply driver 23 inside the lid 21.

The lid 21 is configured to be attachable to and detachable from the housing 11 of the first body portion 10. An upper surface of the lid 21 forms a top surface of the drug solution administration apparatus 100. The electronic control functional components such as the circuit board 22 and the drug solution supply driver 23 are attached to a lower surface side of the lid 21.

The drug solution supply driver 23 includes a motor 23a and the gear 23b. The motor 23a is a drive source for moving the extruding portion 13 in a predetermined direction and supplying the drug solution in the reservoir 12. The gear 23b is constituted by a plurality of stages of gears, and a final-stage gear meshes with the gear 12b. Accordingly, a driving force (a rotational force) from the motor 23a is transmitted to the gear 12b via the gear 23b to drive the extruding portion 13.

In addition, the second body portion 20 includes a supply amount detecting unit 24, such as an encoder, that is capable of detecting a supply amount of the drug solution based on a rotational speed of the motor 23a, a communication unit 25 that is an interface for enabling communication with the outside, and a control unit 26 that is implemented by a known microcomputer including a CPU, a ROM, a RAM, and the like. The communication unit 25 and the drug solution supply driver 23 operate based on predetermined programs under the control of the control unit 26.

### <Cradle>

The cradle 30 includes a cradle body 31 and the connection port 40. The cradle body 31 includes the placement surface portion 31a. The connection port 40 is attached to an upper surface (the placement surface portion 31a) of the cradle body 31.

The cradle body 31 is provided with an adhesive portion 31b on a lower surface thereof. The adhesive portion 31b is a portion that partially and largely protrudes outward from the placement surface portion 31a, and affixes the cradle 30 to the living body surface (the skin of the user). The placement surface portion 31a is formed in a substantially rectangular shape in a plan view.

In the placement surface portion 31a, when the apparatus body 101 is mounted to the cradle 30, the bottom surface portion 101a of the apparatus body 101 (the bottom surface portion 11a of the first body portion 10) is placed on the placement surface portion 31a. The second mounting portions 31c mounted to the first mounting portions 11d of the first body portion 10 are provided on short sides (sides along the Y direction) of the placement surface portion 31a. The second mounting portions 31c are provided with, at tip ends thereof, hook-like engaging protrusions to be fitted into the first mounting portions 11d, and are provided to erect in the thickness direction (the Z direction) at the short sides of the placement surface portion 31a. The second mounting portions 31c are engaged with the first mounting portions 11d in the state where the apparatus body 101 is mounted to the cradle 30.

The placement surface portion 31a includes the first placement surface 31e, the second placement surface 31d, and the second step portion 31f. The first placement surface 31e and the second placement surface 31d are each a plate surface extending along the X direction. The first placement surface 31e is disposed on a mounting direction D1 side of the placement surface portion 31a, and the second placement surface 31d is disposed on a mounting release direction D2 side of the placement surface portion 31a. As illustrated in FIG. 2, the first placement surface 31e and the second placement surface 31d are positioned in a stepped manner in the thickness direction of the cradle body 31 (the Z direction), and the first placement surface 31e is positioned above the second placement surface 31d. When the apparatus body 101 is placed on the cradle 30, the first bottom surface 11e of the bottom surface portion 11a is placed on the first placement surface 31e. When the apparatus body 101 is placed on the cradle 30, the second bottom surface 11f of the bottom surface portion 11a is placed on the second placement surface 31d. In this way, the cradle body 31 includes a plurality of surface portions having different thicknesses from the living body surface.

The second step portion 31f is provided in a boundary portion that defines the first placement surface 31e and the second placement surface 31d. The second step portion 31f extends to vertically cross the cradle body 31 along the X direction. The second step portion 31f has a step surface that rises in a substantially vertical direction (the Z direction) from the second placement surface 31d toward the first placement surface 31e when the cradle 30 is seen from a side surface. When the apparatus body 101 is mounted to the cradle 30, the second step portion 31f functions as an abutting surface that abuts against the first step portion 11g to restrict the rotational movement of the apparatus body 101 in the mounting direction D1.

The placement surface portion 31a may have a shape complementary to that of the bottom surface portion 101a of the apparatus body 101 (the bottom surface portion 11a of the first body portion 10) in the mounting completion state of the apparatus body 101 and the cradle 30. The bottom surface portion 11a is combined with the placement surface portion 31a of the cradle 30 in a complementary manner so as to be integrated with each other in the mounting completion state of the apparatus body 101 and the cradle 30. That is, when the bottom surface portion 11a and the placement surface portion 31a are connected to each other, the shapes of the bottom surface portion 11a and the placement surface portion 31a complement each other across at least one axial direction of the drug solution administration apparatus 100. Therefore, the configuration of the bottom surface portion 11a is not limited to the configuration described above. More specifically, in the state where the mounting of the apparatus body 101 and the cradle 30 is completed, the placement surface portion 31a includes the abutting surface that restricts the movement of the apparatus body 101 in the mounting direction, and the apparatus body 101 has, at the bottom surface portion 11a, a surface facing the abutting surface. The bottom surface portion 11a and the placement surface portion 31a may have a structure at least partially complementary to each other, and preferably, in a plan view of the drug solution administration apparatus 100, shapes complementary to each other are disposed on the D1 side with respect to the engaging recess 17 and the connection port 40, or a corner portion side facing a corner portion where the engaging recess 17 and the connection port 40 are disposed. The bottom surface portion 11a and the placement surface portion 31a may be appropriately provided with a structure such as a switch or a notch necessary for function exhibition and manufacturing of the drug solution administration apparatus 100, in addition to the abutting surface that restricts the movement of the apparatus body 101.

In the drug solution administration apparatus 100, the bottom surface portion 11a and the placement surface portion 31a have shapes complementary to each other. Therefore, when mounting the apparatus body 101 to the cradle 30, a mounting position and a movement direction are easily understood and the mountability is improved in the drug solution administration apparatus 100. In particular, the drug solution administration apparatus 100 is often mounted to the abdomen or the back of the user and is thus useful. Although it is preferable that in the drug solution administration apparatus 100, the bottom surface portion 11a and the placement surface portion 31a have shapes complementary to each other from the viewpoint of improving the mountability, the bottom surface portion 11a and the placement surface portion 31a may not have shapes complementary to each other.

### <Connection Port>

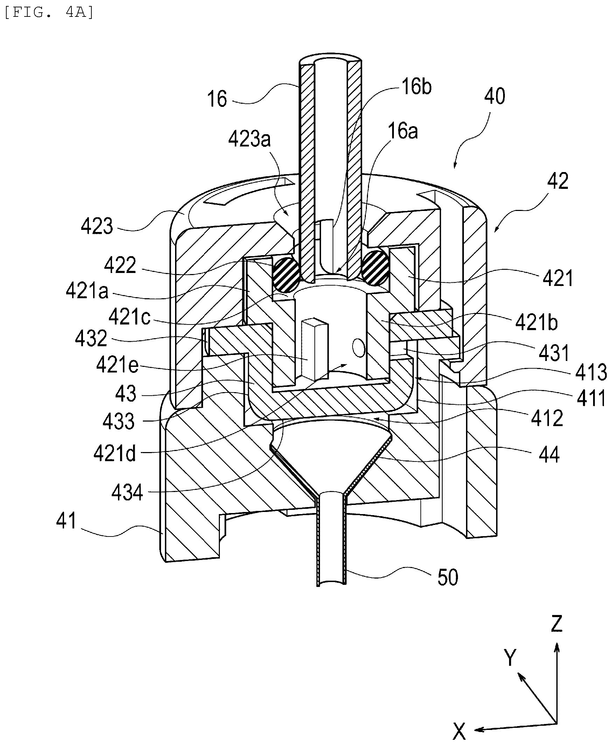

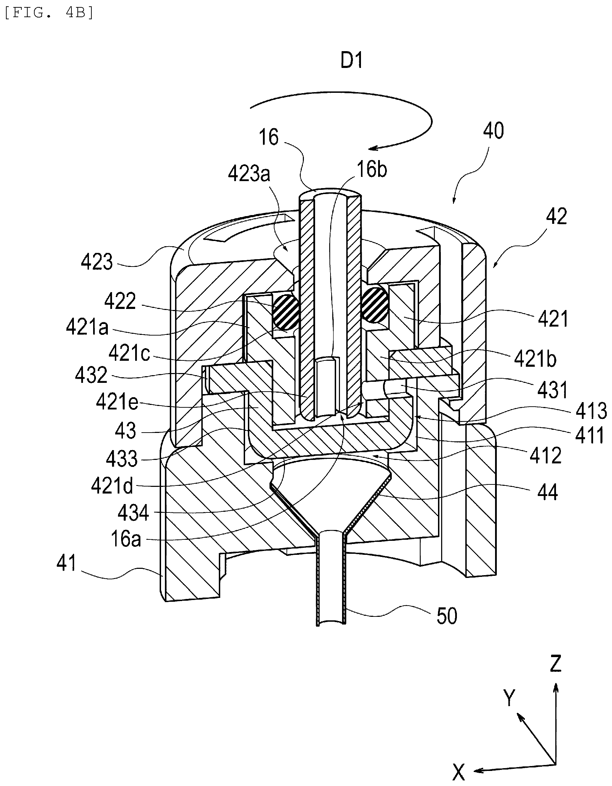

As illustrated in FIG. 4A or FIG. 4B, the connection port 40 is provided on an upper surface of the placement surface portion 31a of the cradle 30. The connection port 40 includes a port body 41, a connection portion 42, the plug 43, and a holding portion 44.

When the apparatus body 101 is mounted to the cradle 30, the connection port 40 is fluidly connected to the drug solution supply portion 16 via the connection portion 42. In the placement surface portion 31a of the cradle 30, the connection port 40 is disposed at a position where the apparatus body 101 is rotationally movable in the state of being temporarily mounted to the cradle 30. Specifically, the connection port 40 is disposed in a vicinity of a corner portion of the placement surface portion 31a of the cradle 30. The connection port 40 is connected to the engaging recess 17 of the apparatus body 101.

The port body 41 is a base portion of the connection port 40 and is locked to the placement surface portion 31a of the cradle 30. The port body 41 includes, at an upper portion, a housing recess 411 in which the rotating member 421 and the plug 43 are housed, and is disposed on the placement surface portion 31a of the cradle 30. The housing recess 411 is a space that has a concave shape with an opening 412 at a substantially central portion of a bottom portion. The housing recess 411, the holding portion 44 positioned below the housing recess 411, the cannula 50, and the opening 412 communicate with one another. A side groove 413 is provided on an inner peripheral surface of the port body 41, and a flow path is formed between an outer surface of the plug 43 and the inner peripheral surface (the opening 412) of the port body 41.

At a lower portion of the port body 41 of the connection port 40, the holding portion 44 that holds a proximal end of the cannula 50 to be indwelled in the living body to supply the drug solution into the living body is provided. The cannula 50 protrudes from the holding portion 44 toward the body of the user, and at least a tip end portion thereof is indwelled in the living body. The cannula 50 is made of a resin material such as a polyurethane, nylon, and an ethylene-tetrafluoroethylene copolymer (ETFE). A puncturing tool (not illustrated) holds a puncturing needle and the connection port 40 holding the cannula 50, and inserts the puncturing needle and the cannula 50 into the living body through a cradle opening 31g. At the same time or subsequently, the connection port 40 is locked to the cradle 30 by a known locking member. After only the puncturing needle is removed from the cradle 30, the puncturing tool is removed from the cradle 30 and is discarded together with the puncturing needle.

The holding portion 44 is formed in a funnel shape so as to guide, to the cannula 50, the drug solution flowing therein when a first communication hole 421d of the rotating member 421 and a second communication hole 431 of the plug 43 are in communication with each other. Therefore, the drug solution flowing into the opening 412 and the holding portion 44 through the first communication hole 421d, the second communication hole 431, and the side groove 413 is introduced into the living body through the cannula 50 along an inner surface of the holding portion 44.

The connection portion 42 is disposed at a position in the upper portion of the port body 41 facing the drug solution supply portion 16. The connection portion 42 and the port body 41 function as an introduction port for introducing the drug solution supplied via the drug solution supply portion 16 into the connection port 40 when the apparatus body 101 is mounted to the cradle 30. Further, the connection portion 42 also functions as a puncturing port to be punctured by the puncturing needle (not illustrated) when causing the cannula 50 to indwell in the living body.

The connection portion 42 includes the rotating member 421, a seal portion 422, and a housing portion 423.

In the housing recess 411, the rotating member 421 is rotatably held in a direction substantially parallel to the placement surface portion 31a with a direction in which a lumen of the cannula 50 extends as a central axis. The rotating member 421 is housed above a recess of the plug 43 on the port body 41. The rotating member 421 is a cylindrical member that has a hollow center having different interior diameters, and includes a large diameter portion 421a and a small diameter portion 421b positioned below the large diameter portion 421a. A step portion 421c protruding from a lower end of the large diameter portion 421a toward the inside of the rotating member 421 (a central axis of the connection portion 42 in the Z direction) is provided between the large diameter portion 421a and the small diameter portion 421b, that is, in a portion where the inner diameter changes in an inner peripheral surface of the large diameter portion 421a. The step portion 421c is continuous with the inner peripheral surface of the large diameter portion 421a and an inner peripheral surface of the small diameter portion 421b, and the inner diameter of the rotating member 421 changes and reduces in the step portion 421c. The seal portion 422 is placed between an inner surface of the housing 423 and the step portion 421c.

The first communication hole 421d is provided on the inner peripheral surface of the small diameter portion 421b of the rotating member 421. When the rotating member 421 is at an open position, the first communication hole 421d communicates with the second communication hole 431 of the plug 43. When the first communication hole 421d and the second communication hole 431 are in communication with each other, the drug solution supplied from the drug solution supply portion 16 can flow to the holding portion 44 through the side groove 413. When the rotating member 421 is at a closed position, the first communication hole 421d is in contact with a side wall 433 of the plug 43 and blocks the communication with the outside. Accordingly, the flow of the drug solution or the like from the first communication hole 421d to a cannula 50 side is blocked by the connection port 40.

The "closed position" described above is a movement position of the rotating member 421 for blocking the fluid connection between the drug solution supply portion 16 and the connection port 40 by closing the first communication hole 421d using the plug 43 in a state where the apparatus body 101 is not mounted to the cradle 30. In addition, the "open position" is a movement position of the rotating member 421 for fluidly connecting the drug solution supply portion 16 and the connection port 40 by opening the first communication hole 421d (that is, a state of communicating with the second communication hole 431) in the state where the apparatus body 101 is mounted to the cradle 30. The closed position and the open position are switched depending on the position of the apparatus body 101 with respect to the cradle 30.

The second engaging portion 421e for engaging with the first engaging portion 16b of the drug solution supply portion 16 is provided on the inner peripheral surface of the small diameter portion 421b of the rotating member 421. The second engaging portion 421e is implemented by, for example, a ridge portion that extends in an axial direction of the inner peripheral surface of the small diameter portion 421b. The second engaging portion 421e is engaged with the first engaging portion 16b when the apparatus body 101 is temporarily mounted to the cradle 30. At this time, in order to secure a flow path having a sufficient size for a flow rate of the drug solution, a tip end surface of the drug solution supply portion 16 may be not in contact with the plug 43. Alternatively, another slit or notch may be provided at the tip end of the drug solution supply portion 16. The second engaging portion 421e is engaged with the first engaging portion 16b when the drug solution supply portion 16 is mounted to the rotating member 421, and the rotational force caused by the rotational movement of the apparatus body 101 in the mounting direction D1 is transmitted to the rotating member 421. Accordingly, the rotating member 421 can rotate following the rotational movement of the apparatus body 101. The second engaging portion 421e can be appropriately designed in accordance with a shape of the first engaging portion 16b so as to be engageable with the first engaging portion 16b.

In the inner peripheral surface of the rotating member 421, disposition positions of the first communication hole 421d and the second engaging portion 421e are set based on a movement amount (a rotational movement distance) of the apparatus body 101 when the apparatus body 101 rotationally moves from the temporarily mounted state of the apparatus body 101 and the cradle 30 in the mounting direction D1 and the mounting thereof is completed. Therefore, when the rotating member 421 is at the closed position, the first communication hole 421d can be provided at a position where the first communication hole 421d comes in contact with the side wall 433 of the plug 43 and is closed. Further, when the rotating member 421 moves from the closed position to the open position, the first communication hole 421d can be provided at a position where the first communication hole 421d communicates with the second communication hole 431.

The rotating member 421 is at the closed position in the state where the apparatus body 101 is not mounted to the cradle 30. As illustrated in FIG. 4B, when the apparatus body 101 is temporarily mounted to the cradle 30, the first engaging portion 16b is engaged with the second engaging portion 421e. In a state where the first engaging portion 16b and the second engaging portion 421e are engaged with each other, the rotating member 421 rotates from the closed position to the open position along with the rotational movement of the apparatus body 101 in the mounting direction D1. Accordingly, since the first communication hole 421d and the second communication hole 431 align and communicate with each other, the drug solution supply portion 16 and the connection port 40 are fluidly connected to each other.

Further, when detaching the apparatus body 101 from the cradle 30, the rotating member 421 rotates from the open position to the closed position along with the rotational movement of the apparatus body 101 in the mounting release direction D2. Accordingly, the first communication hole 421d is closed by the side wall 433 of the plug 43, and the fluid connection between the drug solution supply portion 16 and the connection port 40 is blocked.

The seal portion 422 is in close contact with an outer peripheral surface of the tip end portion of the drug solution supply portion 16 mounted in a mounting hole 423a of the housing portion 423. Therefore, the seal portion 422 can increase liquid tightness in a state where the drug solution supply portion 16 and the housing portion 423 are connected to each other. An O-ring made of a material having flexibility such as a rubber material and a thermoplastic elastomer can be applied as the seal portion 422.

The housing portion 423 is a lid-like member that has a top surface and a peripheral surface and has an open bottom portion, and is attached to the upper portion of the port body 41 so as to cover the rotating member 421. The mounting hole 423a that communicates with the inside of the housing portion 423 is provided in an upper surface (the top surface) of the housing portion 423. The drug solution supply portion 16 is inserted into the mounting hole 423a when the apparatus body 101 is temporarily mounted to the cradle 30. The housing portion 423 may be fixed to and attached to the port body 41, or may be rotatably attached to the port body 41 along with the rotational movement of the apparatus body 101. In addition, the puncturing needle for puncturing and inserting into the cannula 50 can be inserted into the mounting hole 423a.

The plug 43 has a bottomed cylindrical shape in which an upper portion thereof is open in a concave shape to house the rotating member 421, and the side wall 433 surrounding the opening, a bottom portion 434, and a flange portion 432 on an outer periphery of this opening are provided. The plug 43 is housed in the housing recess 411 in a state where the rotating member 421 is housed above the plug 43. As illustrated in FIGS. 4A and 4B, in the state where the plug 43 is housed in the housing recess 411, the flange portion 432 is sandwiched between the port body 41 and the housing portion 423. Accordingly, the plug 43 is positionally fixed (held) in the housing recess 411, and a proximal end of the side groove 413 is sealed. The puncturing needle for puncturing and inserting into the cannula 50 penetrates the bottom portion 434.

The second communication hole 431 is provided on the side wall 433 of the plug 43. The second communication hole 431 is disposed at a position where the second communication hole 431 is communicable with the first communication hole 421d when the rotating member 421 is at the open position.

The plug 43 can be made of a material having flexibility and resealability. Examples of the material having flexibility include various rubber materials such as silicone rubbers and natural rubbers, and various thermoplastic elastomers such as polyurethane-based elastomers, polyester-based elastomers, polyamide-based elastomers, olefin-based elastomers, and styrene-based elastomers. That is, after the puncturing needle that has penetrated the plug 43 is removed from the upper opening of the plug 43, the plug 43 reseals the connection portion 42 to block the communication with the outside.

In the drug solution administration apparatus 100 according to the first embodiment, the apparatus body 101 is caused to rotationally move substantially parallel to the living body surface to be mounted to or separated from the cradle 30 in a state where the engaging recess 17 provided in the apparatus body 101 is engaged with the connection port 40 of the cradle 30. In the drug solution administration apparatus 100 having such a configuration, when the drug solution supply portion 16 is mounted to the connection portion 42, the first engaging portion 16b and the second engaging portion 421e are engaged with each other. Then, when the apparatus body 101 is caused to rotationally move in the mounting direction D1, the rotating member 421 rotates following the rotational movement of the apparatus body 101 and rotationally moves from the closed position to the open position, so that the first communication hole 421d and the second communication hole 431 communicate with each other. That is, in the drug solution administration apparatus 100, a fluid connection state between the drug solution supply portion 16 and the connection port 40 can be switched depending on a relative positional relation between the port body 41 and the rotating member 421 that rotates following the rotational movement of the apparatus body 101.

In the related art, puncturing and removing operations of the drug solution supply needle are repeatedly performed for the rubber plug provided in the connection port. As a result, in the related art, as the number of times of the attachment and detachment of the apparatus body and the cradle increases, for example, there is a risk that a punctured hole of the rubber plug is increased in size or the rubber plug is damaged, and the drug solution leaks from a connection portion between the rubber plug and the drug solution supply needle. In a case where the drug solution leaks during use of the drug solution administration apparatus, particularly in a case where a dosage is a trace amount, the patient is likely to be aware of the leakage with a delay, which is thus not preferable. In contrast, in the drug solution administration apparatus 100 according to the first embodiment, the drug solution supply portion 16 having the cylindrical hollow shape is mounted to the mounting hole 423a of the housing portion 423, and the fluid connection state between the drug solution supply portion 16 and the connection port 40 can be switched depending on the relative positional relation between the rotating member 421 and the port body 41. Therefore, the drug solution administration apparatus 100 can have improved durability as compared with that in the related art. In addition, since the drug solution supply portion 16 and the connection port 40 are fluidly connected to each other only when the rotating member 421 is at the open position, the drug solution administration apparatus 100 can block the communication with the outside in the state where the apparatus body 101 is not mounted to the cradle 30.

Further, in the drug solution administration apparatus 100, the rotating member 421 rotates following the rotational movement of the apparatus body 101 in the mounting direction D1 substantially parallel to the placement surface portion 31a in the state where the drug solution supply portion 16 is mounted. Therefore, when mounting the apparatus body 101 to the cradle 30, the rotating member 421 becomes the rotation center and a rotating direction of the apparatus body 101 is guided, so that the user can stably perform the mounting operation.

### <Operations>

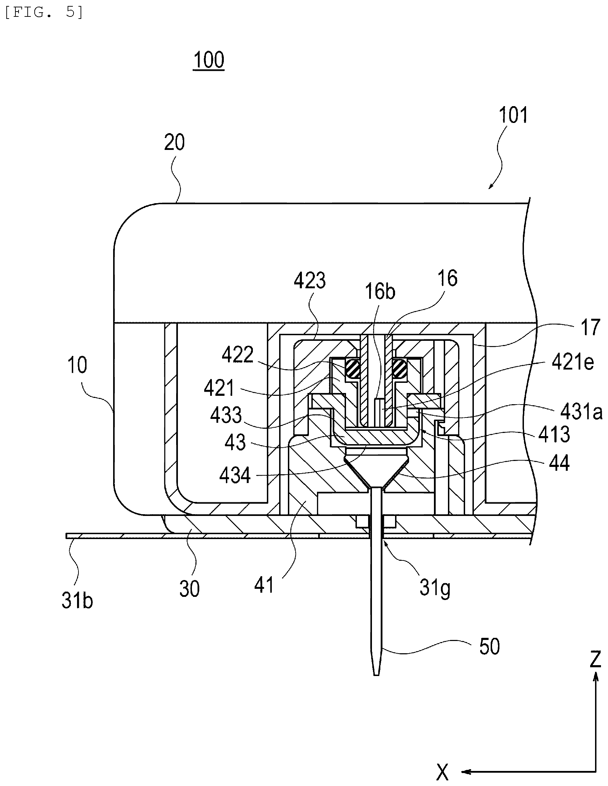

Next, operations when mounting the apparatus body 101 to the cradle 30 in the drug solution administration apparatus 100 according to the first embodiment will be described with reference to FIGS. 5 to 8.

As a stage before using the drug solution administration apparatus 100, the user mounts the cradle 30 to the living body surface, and indwells the cannula 50 in the living body. When indwelling the cannula 50, the user inserts the cannula 50 into the living body together with the connection portion 42 by using a puncturing tool (not illustrated) while adhering the adhesive portion 31b of the cradle 30 to the skin. The cannula 50 is inserted into the living body by using a puncturing needle. The user removes the puncturing needle and the puncturing tool (not illustrated) used for the insertion of the puncturing needle from the cradle 30 in a state where the cannula 50 is indwelled in the living body.

Next, the user assembles the apparatus body 101 by connecting the second body portion 20 to the first body portion 10. The user lowers the assembled apparatus body 101 in the Z direction to the cradle 30 so as to engage the engaging recess 17 with the connection port 40. Accordingly, as illustrated in FIG. 5, the apparatus body 101 is temporarily mounted in the state where the engaging recess 17 of the apparatus body 101 is engaged with the connection port 40. Further, in the state where the apparatus body 101 and the cradle 30 are mounted to each other, the drug solution supply portion 16 is inserted into the mounting hole 423a of the housing portion 423, and the first engaging portion 16b and the second engaging portion 421e are engaged with each other. The rotating member 421 is at the closed position in a stage before the apparatus body 101 rotationally moves in the mounting direction D1.

As illustrated in FIG. 6, the user causes the apparatus body 101 to rotationally move in the mounting direction D1, and then engages the first mounting portions 11d with the second mounting portions 31c. At this time, the user causes the apparatus body 101 to rotationally move to a position where the first step portion 11g on the bottom surface portion 101a of the apparatus body 101 and the second step portion 31f of the cradle 30 come in contact with each other. Accordingly, as illustrated in FIG. 6, the drug solution administration apparatus 100 is in a mounting completion state.

Further, as illustrated in FIG. 6, in an engaged state between the first engaging portion 16b and the second engaging portion 421e, the rotating member 421 rotates from the closed position to the open position along with the rotational movement of the apparatus body 101 in the mounting direction D1. Accordingly, the first communication hole 421d and the second communication hole 431 communicate with each other, and a drug solution flow path (the side groove 413 that is a flow path connected to the cannula 50 in the connection port 40) communicating with the drug solution supply portion 16 is provided. The drug solution supply portion 16 and the connection port 40 are fluidly connected to each other, and the drug solution can be administered by the drug solution administration apparatus 100. In this state, when the user starts the drug solution administration apparatus 100, the drug solution supply driver 23 drives the drug solution stored in the reservoir 12 at a predetermined timing to flow from the drug solution supply portion 16 to the port body 41 through the drug solution supply pipe 14. The drug solution that has flowed into the port body 41 by the start of the drug solution administration apparatus 100 is introduced into the living body through the cannula 50.

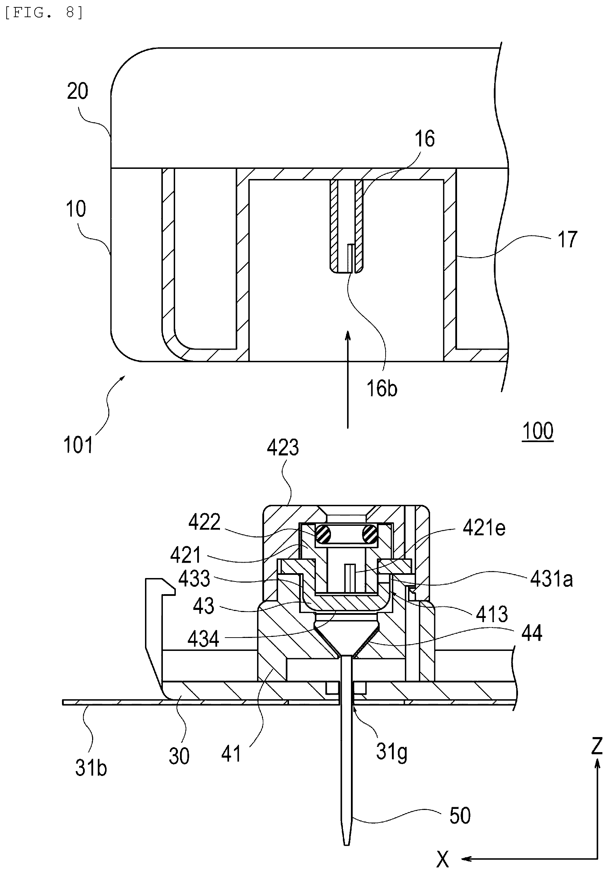

When detaching the apparatus body 101 from the cradle 30 during the use of the drug solution administration apparatus 100, as illustrated in FIG. 7, the user releases the engagement between the first mounting portions 11d and the second mounting portions 31c, and then causes the apparatus body 101 to rotationally move in the mounting release direction D2 with the engaging position between the engaging recess 17 and the connection port 40 as the rotation center. Accordingly, a part of the bottom surface portion 101a of the apparatus body 101 is separated from the placement surface portion 31a of the cradle 30. In addition, the rotating member 421 rotates from the open position to the closed position along with the rotational movement of the apparatus body 101 in the mounting release direction D2. Accordingly, the first communication hole 421d is closed by the side wall 433 of the plug 43, and the fluid connection between the drug solution supply portion 16 and the connection port 40 is blocked. Then, as illustrated in FIG. 8, the user moves the apparatus body 101 upward. Accordingly, the apparatus body 101 is completely detached from the cradle 30. At this time, since the rotating member 421 is at the closed position, the first communication hole 421d is in a closed state, and the communication with the outside of the connection port 40 is blocked.

### [Second Embodiment]

Next, the drug solution administration apparatus 110 according to a second embodiment will be described with reference to FIGS. 9 to 13. In the second embodiment, components having the same functions as those of the first embodiment described above are denoted by the same reference numerals, and detailed descriptions thereof are omitted, and for example, a configuration, members, and a using method may be the same as those of the embodiment described above unless otherwise specified.

The second embodiment to be described below describes a configuration in which a shape of an engaging recess 17a provided in a bottom surface portion 111a of an apparatus body 111 and the shape of the connection port 40 are changed.

### <Configuration>

In the drug solution administration apparatus 110 according to the second embodiment, a rotating member 424 is rotatably mounted to an outer peripheral surface of the upper portion of the port body 41 of the connection port 40. The rotating member 424 is engaged with the engaging recess 17a when the apparatus body 111 is temporarily mounted to the cradle 30. In addition, the drug solution administration apparatus 110 according to the second embodiment includes a needle-like drug solution supply portion 19 instead of the drug solution supply portion 16 having the cylindrical hollow shape.

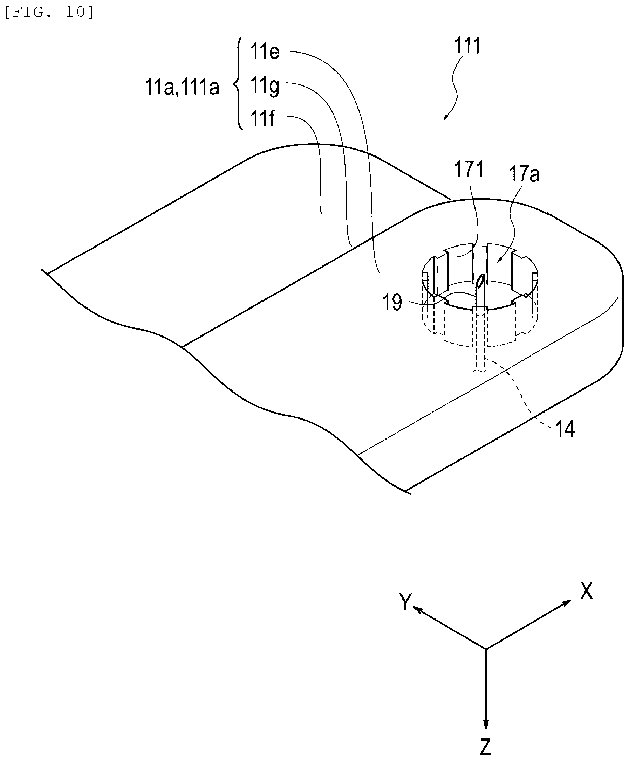

The drug solution supply portion 19 is a needle member having a hollow needle tube and a sharp puncturing end. A proximal end of the drug solution supply portion 19 is connected to the drug solution supply pipe 14, and the puncturing end at a tip end is caused to penetrate and puncture the plug 43 of the connection port 40 provided in the cradle 30. As illustrated in FIG. 10, the drug solution supply portion 19 is disposed in a manner of not protruding from the engaging recess 17a inside the engaging recess 17a provided in the bottom surface portion 11a of the first body portion 10.

The engaging recess 17a is provided to be recessed in the thickness direction of the housing 11 on the bottom surface portion 11a (specifically, the first bottom surface 11e) of the first body portion 10. An inner peripheral surface 171 of the engaging recess 17a has a concave-convex shape in which protrusions and recesses formed in a radial direction from an axial center (an extending direction of the drug solution supply portion 19) are alternately disposed along a circumferential direction. The inner peripheral surface 171 of the engaging recess 17a is engaged with an outer peripheral surface 424d of the rotating member 424. The drug solution supply portion 19 is disposed at a substantially central portion of the engaging recess 17a.

The port body 41 is locked to the placement surface portion 31a of the cradle 30. The port body 41 holds the rotating member 424 in a manner of being rotationally rotatable at the axial center. A seal portion 425 is disposed between the port body 41 and the rotating member 424. The seal portion 425 seals a gap between the rotating member 424 and the port body 41 to hermetically close the port body 41. Similar to the seal portion 422, an O-ring made of a material having flexibility and sealability such as a rubber material and a thermoplastic elastomer can be applied as the seal portion 425.

The rotating member 424 has a cylindrical hollow shape in which a housing portion 424a is provided. The rotating member 424 includes a hollow shaft portion 424c extending downward from the housing portion 424a.

The housing portion 424a has a concave shape that is provided with, at a substantially central portion of a bottom portion, an opening 424b communicating with a lumen of the shaft portion 424c, and houses the plug 43. The opening 424b of the housing portion 424a communicates with the holding portion 44 via the lumen of the shaft portion 424c. The rotating member 424 includes the shaft portion 424c having a cylindrical hollow shape that extends downward from the bottom portion of the housing portion 424a. The shaft portion 424c is housed in the housing recess 411 provided on a base portion of the port body 41.

The outer peripheral surface 424d of the rotating member 424 has a concave-convex shape in which protrusions and recesses formed in a radial direction from an axial center are alternately disposed along a circumferential direction. The outer peripheral surface 424d of the rotating member 424 is engaged with the inner peripheral surface 171 of the engaging recess 17a. That is, an inner peripheral shape of the engaging recess 17a and an outer peripheral shape of the connection port 40 are shapes complementary to each other. Since it is sufficient that the inner peripheral surface 171 of the engaging recess 17a and the outer peripheral surface 424d of the rotating member 424 have shapes complementary to each other, shapes of the protrusions and the recesses, a disposition interval in the circumferential direction, and the like are not particularly limited.

The rotating member 424 is attached to the port body 41 rotatably in the mounting direction D1 and the mounting release direction D2 (see FIG. 9). When the apparatus body 111 is caused to rotationally move in the mounting direction D1 after the apparatus body 111 is temporarily mounted to the cradle 30, the rotating member 424 rotates along with the movement while engaging with the engaging recess 17a. Specifically, the rotating member 424 rotates in a state of being engaged with the engaging recess 17a with an engaging position between the engaging recess 17a and the rotating member 424 (specifically, a puncturing position of the drug solution supply portion 19 to the plug 43) as a rotation center in a state where the drug solution supply portion 19 is punctured. Further, when the apparatus body 111 is caused to rotationally move in the mounting release direction D2 in order to detach the apparatus body 111 from the cradle 30, the rotating member 424 rotates along with the movement while being engaged with the engaging recess 17a.

In the second embodiment, the plug 43 is subjected to puncturing and removing operations by both the puncturing needle and the drug solution supply portion 19. After the puncturing needle that has penetrated the plug 43 is removed, the plug 43 seals the connection portion 42 to block the communication with the outside. In addition, after the drug solution supply portion 19 that has penetrated the plug 43 is removed, the plug 43 seals the connection portion 42 to block the communication with the outside.

Similar to the drug solution administration apparatus 100 according to the first embodiment, in the drug solution administration apparatus 110 according to the second embodiment, the apparatus body 111 is caused to rotationally move to be mounted to or separated from the cradle 30 in a state where the engaging recess 17a provided in the apparatus body 111 is engaged with the connection port 40 of the cradle 30. In the drug solution administration apparatus 110 having such a configuration, the rotating member 424 is rotatably attached to the port body 41, and the plug 43 is disposed inside the rotating member 424. In the drug solution administration apparatus 110, when the apparatus body 111 is temporarily mounted to the cradle 30, the drug solution supply portion 19 is caused to puncture and be mounted to the plug 43. In the drug solution administration apparatus 110, when the apparatus body 111 is caused to rotationally move in the mounting direction D1 after the apparatus body 111 is temporarily mounted to the cradle 30, the rotating member 424 rotates following the movement in the state where the rotating member 424 is engaged with the engaging recess 17a with the engaging position between the engaging recess 17a and the rotating member 424 (the puncturing position of the drug solution supply portion 19 to the plug 43) as the rotation center.

Therefore, in the drug solution administration apparatus 110, when mounting the apparatus body 111 and the cradle 30 to each other, it is possible to cause the apparatus body 111 to rotationally move without deviating a puncturing posture of the drug solution supply portion 19 in a height direction or a lateral direction while the drug solution supply portion 19 is in a state of puncturing the plug 43. Therefore, the drug solution administration apparatus 110 can reduce the damage to the plug 43 by the drug solution supply portion 19. In addition, in the drug solution administration apparatus 110, when mounting the apparatus body 111 to the cradle 30, the drug solution supply portion 19 is caused to puncture the plug 43 in a state where the engaging recess 17a is guided by the rotating member 424, so that a puncturing portion of the drug solution supply portion 19 to the plug 43 is less likely to be scattered, and the deterioration due to the damage to the plug 43 can be reduced. Further, when mounting the apparatus body 111 on the cradle 30, the rotating member 424 becomes the rotation center and a rotating direction of the apparatus body 111 is guided, so that the user can stably perform the mounting operation without moving the drug solution supply portion 19 during a rotating operation.

### <Operations>

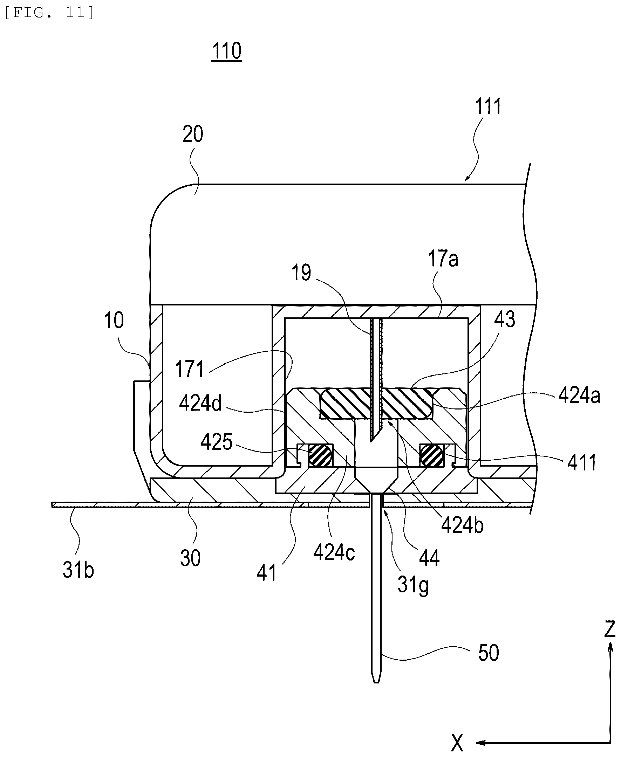

Next, operations when mounting the apparatus body 111 to the cradle 30 in the drug solution administration apparatus 110 according to the second embodiment will be described with reference to FIGS. 11 to 13.

As a stage before using the drug solution administration apparatus 110, the user mounts the cradle 30 to the living body surface, and indwells the cannula 50 in the living body. When indwelling the cannula 50, the user punctures a puncturing needle and the cannula 50 through the cradle opening 31g, and inserts only the cannula 50 into the living body while adhering the adhesive portion 31b of the cradle 30 to the skin. The user removes the puncturing needle and a puncturing tool (not illustrated) used for the insertion of the puncturing needle from the connection portion 42 and the cradle 30 in the state where the cannula 50 is indwelled in the living body.

Next, the user assembles the apparatus body 111 by connecting the second body portion 20 to the first body portion 10. The user lowers the assembled apparatus body 111 in the Z direction to the cradle 30 so as to engage the engaging recess 17a with the rotating member 424 of the connection port 40. Accordingly, as illustrated in FIG. 11, the apparatus body 111 is temporarily mounted in a state where the engaging recess 17a of the apparatus body 111 is engaged with the rotating member 424 of the connection port 40. At this time, the drug solution supply portion 19 penetrates and is mounted to the plug 43. Accordingly, in the connection port 40, a drug solution flow path communicating with the drug solution supply portion 19 (the flow path connected to the cannula 50 in the connection port 40) is formed, and the drug solution can be administered by the drug solution administration apparatus 110. That is, the connection port 40 is in a state of being fluidly connected to the drug solution supply portion 19. However, the mounting of the apparatus body 111 to the cradle 30 has not yet been completed.

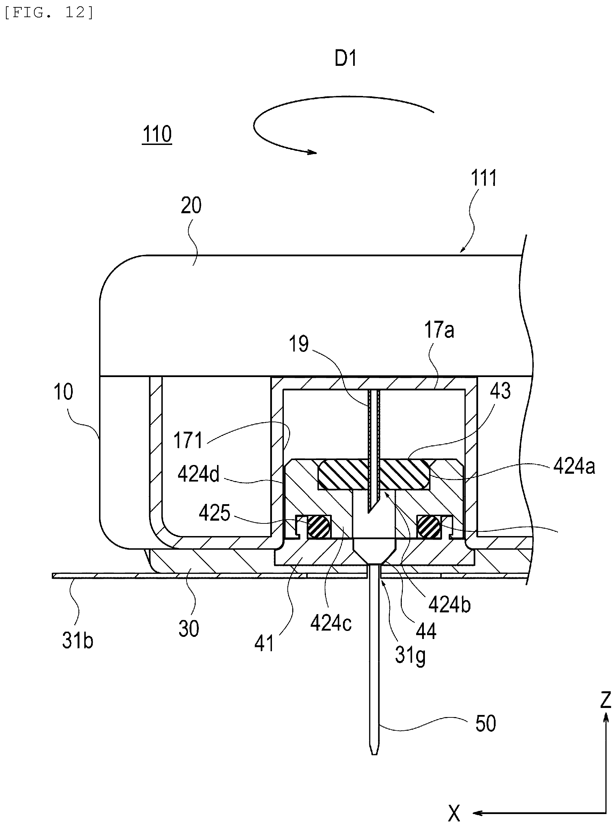

As illustrated in FIG. 12, the user causes the apparatus body 111 to rotationally move in the mounting direction D1, and then engages the first mounting portions 11d with the second mounting portions 31c. At this time, the user causes the apparatus body 111 to rotationally move to a position where the first step portion 11g on the bottom surface portion 111a of the apparatus body 111 and the second step portion 31f of the cradle 30 come in contact with each other. In addition, the rotating member 424 rotates following the engaging recess 17a with the engaging position between the engaging recess 17a and the rotating member 424 as the rotation center in the state where the rotating member 424 is engaged with the engaging recess 17a. At this time, the drug solution supply portion 19 is in the state of puncturing the plug 43, and a puncturing posture during the puncturing is maintained due to the rotation of the rotating member 424.

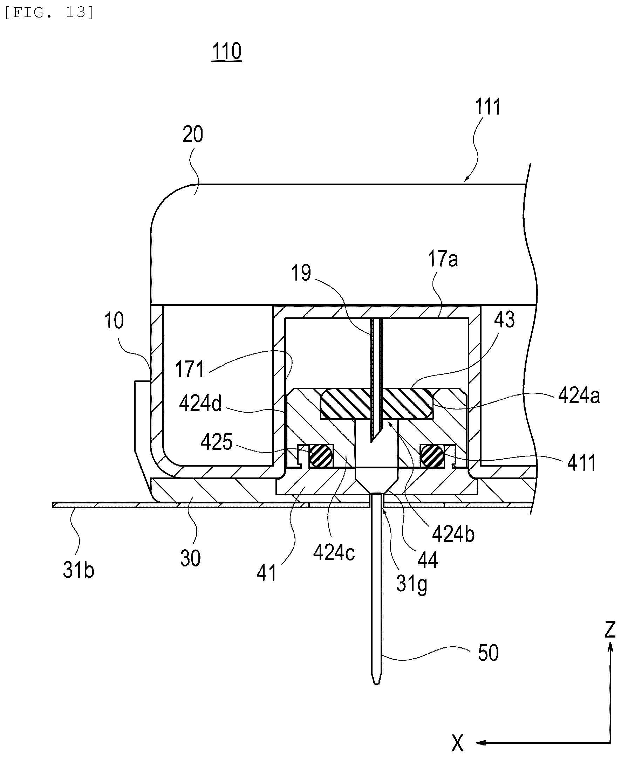

After causing the apparatus body 111 to rotationally move in the mounting direction D1, the user engages the first mounting portions 11d with the second mounting portions 31c. Accordingly, the apparatus body 111 is completely placed on the cradle 30, and the mounting for the drug solution administration apparatus 110 is completed as illustrated in FIG. 13. In the state where the mounting of the apparatus body 111 to the cradle 30 is completed, when the user starts the drug solution administration apparatus 110, the drug solution supply driver 23 drives the drug solution stored in the reservoir 12 at a predetermined timing to flow from the drug solution supply portion 19 to the port body 41 through the drug solution supply pipe 14. The drug solution that has flowed into the port body 41 by the start of the drug solution administration apparatus 110 is introduced into the living body through the cannula 50.

When detaching the apparatus body 111 from the cradle 30 during the use of the drug solution administration apparatus 110, the user releases the engagement between the first mounting portions 11d and the second mounting portions 31c, and then causes the apparatus body 111 to rotationally move in the mounting release direction D2 with the engaging position between the engaging recess 17a and the connection port 40 as the rotation center. Accordingly, a part of the bottom surface portion 111a of the apparatus body 111 is separated from the placement surface portion 31a of the cradle 30. In addition, the rotating member 424 rotates following the rotational movement of the apparatus body 111 in the mounting release direction D2. Then, the user moves the apparatus body 111 upward. Accordingly, the apparatus body 111 is completely detached from the cradle 30. After the drug solution supply portion 19 that has penetrated the plug 43 is removed, the connection port 40 seals the connection portion 42 to block the communication with the outside.

### <Functions and Effects>

As described above, the drug solution administration apparatus 100, 110 according to the embodiments include: the apparatus body 101, 111 that includes the reservoir 12 configured to store the drug solution, the drug solution supply driver 23 configured to supply the drug solution from the reservoir 12, the drug solution supply pipe 14 through which the drug solution supplied from the reservoir 12 flows, and the drug solution supply portion 16, 19 communicating with the drug solution supply pipe 14; and the cradle that includes the placement surface portion 31a on which the bottom surface portion 101a, 111a (the bottom surface portion 11a) of the apparatus body 101, 111 is placed, and the connection port 40 disposed on the upper surface of the placement surface portion 31a and configured to hold the cannula 50 to be inserted into the living body, and that has a living body side adhered to the living body with respect to the apparatus body 101, 111, in which the drug solution supply portion 16, 19 and the connection port 40 are fluidly connected to each other in a detachable manner. In the drug solution administration apparatus 100, 110 having such a configuration, the connection port 40 includes the port body 41 configured to hold the cannula 50 and be locked to the cradle 30, and the rotating member 421, 424 to which the drug solution supply portion 16, 19 is mounted, and that is rotatable, with the direction in which the lumen of the cannula 50 extends as the central axis, in the direction substantially parallel to the placement surface portion 31a, and the rotating member 421, 424 rotates following the rotational movement of the apparatus body 101, 111 substantially parallel to the placement surface portion 31a in the state where the drug solution supply portion 16, 19 is mounted.

According to the drug solution administration apparatus 100, 110 configured as described above, when mounting the apparatus body 101, 111 to the cradle 30, the rotating direction of the apparatus body 101, 111 is guided with the rotating member 421, 424 as the rotation center, so that the user can stably perform the mounting operation, and the mounting posture of the drug solution supply portion 16, 19 does not deviate in the height direction or the lateral direction. For example, in a case of a configuration in which a needle member having a needle tube is adopted as the drug solution supply portion 19, and is connected to the plug 43 provided in the connection port 40 by puncturing, such as the drug solution administration apparatus 110 according to the second embodiment, the puncturing position of the drug solution supply portion 19 to the plug 43 is less likely to deviate during the rotational movement of the apparatus body 111. Therefore, in the drug solution administration apparatus 110, the increase in size of the punctured hole of the plug 43 is restrained, and the damage caused by puncturing damages having different orientations with respect to the thickness direction of the plug 43 due to the repetition of the puncturing and removing operations, and the decrease in the sealability of the plug 43 are reduced.

Further, the drug solution administration apparatus 100 according to the first embodiment may have a configuration in which the drug solution supply portion 16 has the cylindrical hollow shape, the rotating member 421 has the first communication hole 421d allowing the drug solution introduced from the drug solution supply portion 16 to flow therethrough, the connection port 40 includes the plug 43 having the second communication hole 431 communicable with the first communication hole 421d and disposed on the outer peripheral side of the rotating member 421, and the connection port 40 rotates following the rotational movement of the apparatus body 101 in the mounting direction D1 substantially parallel to the placement surface portion 31a when the apparatus body 101 is mounted to the cradle 30, or in the mounting release direction D2 that is a direction opposite to the mounting direction D1, and the fluid connection state between the connection port 40 and the drug solution supply portion 16 is switched depending on the relative positional relation with the connection port 40.

In the drug solution administration apparatus 100 configured as described above, the drug solution supply portion 16 having a cylindrical hollow shape is mounted to the connection port 40 (the rotating member 421), and the fluid connection state between the drug solution supply portion 16 and the connection port 40 can be switched depending on the relative positional relation between the rotating member 421 and the port body 41. Since a needle is not used in the drug solution supply portion 16, the drug solution administration apparatus 100 can avoid a problem that the drug solution leaks from the connection port due to the damage and deterioration of the rubber plug. Further, the user can switch the fluid connection state between the drug solution supply portion 16 and the connection port 40 simply by causing the apparatus body 101 to rotationally move in the mounting direction D1 or the mounting release direction D2. In this way, switching of the flow path in conjunction with a connection operation of the apparatus body 101 can be achieved.

The drug solution administration apparatus 100 according to the first embodiment may have a configuration in which the rotating member 421 is rotatably attached to the port body 41 between the closed position where the first communication hole 421d is closed and the open position where the first communication hole 421d is open, and the rotating member 421 moves form the closed position to the open position by rotating following the rotational movement of the apparatus body 101 in the mounting direction D1 in the state where the drug solution supply portion 16 is mounted, whereby the first communication hole 421d and the second communication hole 431 communicate with each other and the drug solution supply portion 16 and the connection port 40 are fluidly connected to each other.

According to the drug solution administration apparatus 100 configured as described above, the user simply mounts the drug solution supply portion 16 to the rotating member 421 and causes the apparatus body 101 to rotationally move in the mounting direction D1, whereby the rotating member 421 rotates following the rotational movement and moves from the closed position to the open position, the first communication hole 421d and the second communication hole 431 communicate with each other, and the drug solution supply portion 16 and the connection port 40 are fluidly connected each other. In addition, since the drug solution supply portion 16 and the connection port 40 are fluidly connected to each other only when the rotating member 421 is at the open position, the drug solution administration apparatus 100 can block the communication with the outside in the state where the apparatus body 101 is not mounted to the cradle 30.

Further, the drug solution administration apparatus 100 according to the first embodiment may have a configuration in which the drug solution supply portion 16 includes the first engaging portion 16b at the tip end thereof, the rotating member 421 includes the second engaging portion 421e engageable with the first engaging portion 16b, and the first engaging portion 16b is engaged with the second engaging portion 421e when the drug solution supply portion 16 is mounted to the rotating member 421, and transmits the rotational force caused by the rotational movement of the apparatus body 101 in the mounting direction D1 to the rotating member 421 via the second engaging portion 421e.

According to the drug solution administration apparatus 100 configured as described above, the first engaging portion 16b and the second engaging portion 421e are engaged with each other, whereby the rotational force caused by the rotational movement of the apparatus body 101 can be transmitted to the rotating member 421. Therefore, the rotating member 421 can be caused to rotate from the closed position to the open position by a mounting operation of mounting the apparatus body 101 to the cradle 30. Therefore, when mounting the apparatus body 101 to the cradle 30, the drug solution administration apparatus 100 can easily switch the fluid connection state between the drug solution supply portion 16 and the connection port 40.

Further, the drug solution administration apparatus 100, 110 according to the embodiments may have a configuration in which the rotating member 421, 424 includes, inside the rotating member 421, 424, the seal portion 422, 43 that is in close contact with the outer peripheral surface of the mounted drug solution supply portion 16, 19.

According to the drug solution administration apparatus 100, 110 configured as described above, since the seal portion 422, 43 is in close contact with the outer periphery of the mounted drug solution supply portion 16, 19, the liquid tightness in the connection state between the drug solution supply portion 16, 19 and the rotating member 421 can be increased.

In addition, the drug solution administration apparatus 110 according to the second embodiment may have a configuration in which the rotating member 421 is provided on the outer peripheral surface 424d of the connection port 40.

According to the drug solution administration apparatus 110 configured as described above, the apparatus body 111 is stably mounted to the cradle 30 while maintaining the fluid connection between the drug solution supply portion 19 and the plug 43.

Further, the drug solution administration apparatus 110 according to the second embodiment may have a configuration in which the drug solution supply portion 19 is implemented by a needle member having a needle tube, the apparatus body 111 includes, at the bottom surface portion 111a (11a), the engaging recess 17a that is provided with the drug solution supply portion 19 therein and that is engageable with the rotating member 424, and the rotating member 424 includes the plug 43 where the drug solution supply portion 19 penetrates and is mounted, and rotates following the rotational movement of the apparatus body 111 in the mounting direction D1 substantially parallel to the placement surface portion 31a in the state where the drug solution supply portion 19 is mounted to the plug 43, or in the mounting release direction D2 that is a direction opposite to the mounting direction D1.

According to the drug solution administration apparatus 110 configured as described above, when mounting the apparatus body 111 to the cradle 30, the apparatus body 111 can be caused to rotationally move without deviating the puncturing posture of the drug solution supply portion 19 in the height direction or the lateral direction in the state where the plug 43 is punctured even by the needle-like drug solution supply portion 19. Therefore, the drug solution administration apparatus 110 can reduce the damage to the plug 43 by the drug solution supply portion 19.

In addition, the drug solution administration apparatus 100, 110 according to the embodiments may have a configuration in which the apparatus body 101, 111 includes the first mounting portions 11d on the side surfaces, and the cradle 30 includes the second mounting portions 31c that are engaged with the first mounting portions 11d.

According to the drug solution administration apparatus 100, 110 configured as described above, when mounting the apparatus body 101, 111 to the cradle 30, the first mounting portions 11d are engaged with the second mounting portions 31c so as to stably maintain the mounted state, and thus the drug solution administration apparatus 100, 110 can be safely used without the first mounting portions 11d being separated from the second mounting portions 31c during use.

The application is based on Japanese Patent Application No. 2021-050938 filed on March 25, 2021.

### Reference Signs List

10 first body portion
11 housing (11a bottom surface portion, 11d first mounting portion)
12 reservoir
14 drug solution supply pipe
16 drug solution supply portion having cylindrical hollow shape (16a drug solution supply hole, 16b first engaging portion)
17, 17a engaging recess
19 drug solution supply portion having needle tube
20 second body portion
23 drug solution supply driver (23a motor, 23b gear)
30 cradle
31 cradle body (31a placement surface portion, 31c second mounting portion)
40 connection port
42 connection portion (41 port body, 421, 424 rotating member, 421d first communication hole, 421e second engaging portion, 422 seal portion, 424d outer peripheral surface)
43 plug (431 second communication hole)
50 cannula
100, 110 drug solution administration apparatus
101, 111 apparatus body (101a, 111a bottom surface portion)
D1 mounting direction
D2 mounting release direction.

## Claims

1. A drug solution administration apparatus (100, 110), comprising:
an apparatus body (101, 111) that includes a reservoir (12) configured to store a drug solution, a drug solution supply driver (23) configured to supply the drug solution from the reservoir (12), a drug solution supply pipe (14) through which the drug solution supplied from the reservoir (12) flows, and a drug solution supply portion (16, 19) communicating with the drug solution supply pipe (14); and
a cradle (30) that includes a placement surface portion (31a) on which a bottom surface portion (11a) of the apparatus body (101, 111) is placed, and a connection port (40) disposed on an upper surface of the placement surface portion (31a) and configured to hold a cannula (50) to be inserted into a living body, and that has a living body side adhered to the living body with respect to the apparatus body (101, 111),
wherein the apparatus body (101, 111) includes a first mounting portion (11d) on a side surface, and
the cradle (30) includes a second mounting portion (31c) that is engaged with the first mounting portion (11d), and
wherein
the drug solution supply portion (16, 19) and the connection port (40) are fluidly connected to each other in a detachable manner,
the connection port (40) includes
a port body (41) configured to hold the cannula (50) and be locked to the cradle (30),
**characterized in that**
the connection port (40) further includes
a rotating member (421, 424) to which the drug solution supply portion (16, 19) is mounted, and that is rotatable, with a direction in which a lumen of the cannula (50) extends as a central axis, in a direction substantially parallel to the placement surface portion (31a), and
the rotating member (421, 424) rotates following a rotational movement of the apparatus body (101, 111) in a mounting direction (D1) substantially parallel to the placement surface portion (31a) in a state where the drug solution supply portion (16, 19) is mounted.

2. The drug solution administration apparatus (100, 110) according to claim 1, wherein
the drug solution supply portion (16, 19) has a cylindrical hollow shape,
the rotating member (421, 424) has a first communication hole (421d) allowing the drug solution introduced from the drug solution supply portion (16, 19) to flow therethrough,
the connection port (40) includes a plug (43) having a second communication hole (431) communicable with the first communication hole (421d) and disposed on an outer peripheral side of the rotating member (421, 424), and
the connection port (40) rotates following a rotational movement of the apparatus body (101, 111) in the mounting direction (D1) substantially parallel to the placement surface portion (31a) when the apparatus body (101, 111) is mounted to the cradle (30), or in a mounting release direction (D2) that is a direction opposite to the mounting direction (D1), and a fluid connection state between the connection port (40) and the drug solution supply portion (16, 19) is switched depending on a relative positional relation between the port body (41) and the connection port (40).

3. The drug solution administration apparatus (100, 110) according to claim 2, wherein
the rotating member (421, 424) is rotatably attached to the port body (41) between a closed position where the first communication hole (421d) is closed and an open position where the first communication hole (421d) is open, and
the rotating member (421, 424) moves form the closed position to the open position by rotating following the rotational movement of the apparatus body (101, 111) in the mounting direction (D1) in the state where the drug solution supply portion (16, 19) is mounted, whereby the first communication hole (421d) and the second communication hole (431) communicate with each other and the drug solution supply portion (16, 19) and the connection port (40) are fluidly connected to each other.

4. The drug solution administration apparatus (100, 110) according to claim 2 or 3, wherein
the drug solution supply portion (16, 19) includes a first engaging portion (16b) at a tip end thereof,
the rotating member (421, 424) includes a second engaging portion (421e) engageable with the first engaging portion (16b), and
the first engaging portion (16b) is engaged with the second engaging portion (421e) when the drug solution supply portion (16, 19) is mounted to the rotating member (421, 424), and transmits a rotational force caused by the rotational movement of the apparatus body (101, 111) in the mounting direction (D1) to the rotating member (421, 424) via the second engaging portion (421e).

5. The drug solution administration apparatus (100, 110) according to any one of claims 2 to 4, wherein
the rotating member (421, 424) includes, inside the rotating member (421, 424), a seal portion (422) that is in close contact with an outer peripheral surface of the mounted drug solution supply portion (16, 19).

6. The drug solution administration apparatus (100, 110) according to claim 1, wherein
the rotating member (421, 424) is provided on an outer peripheral surface of the connection port (40).

7. The drug solution administration apparatus (100, 110) according to claim 6, wherein
the drug solution supply portion (16, 19) is implemented by a needle member (19) having a needle tube,
the apparatus body (101, 111) includes, at the bottom surface portion (11a), an engaging recess (17a) that is provided with the drug solution supply portion (16, 19) therein and that is engageable with the rotating member (421, 424), and
the rotating member (421, 424) includes a plug (43) where the drug solution supply portion (16, 19) penetrates and is mounted, and rotates following the rotational movement of the apparatus body (101, 111) in the mounting direction (D1) in a state where the drug solution supply portion (16, 19) is mounted to the plug (43).

## Patentansprüche

1. Medikamentenlösungshandhabungsvorrichtung (100, 110) mit:
einem Vorrichtungskörper (101, 111), der einen Speicher (12), der so aufgebaut ist, dass er eine Medikamentenlösung speichert, eine Medikamentenlösungslieferantriebseinrichtung (23), die so aufgebaut ist, dass sie die Medikamentenlösung von dem Speicher (12) liefert, ein Medikamentenlösungslieferrohr (14), durch das die von dem Speicher (12) gelieferte Medikamentenlösung strömt, und einen Medikamentenlösungslieferabschnitt (16, 19) aufweist, der mit dem Medikamentenlösungslieferungsrohr (14) in Kommunikation steht; und
einem Träger (30), der einen Anordnungsflächenabschnitt (31a), auf dem ein Bodenflächenabschnitt (11a) des Vorrichtungskörpers (101, 111) angeordnet ist, und einen Verbindungsanschluss (40) aufweist, der an einer oberen Fläche des Anordnungsflächenabschnittes (31a) angeordnet ist und so aufgebaut ist, dass er eine in einen lebenden Körper einzuführende Kanüle (50) hält, und der eine Lebendkörperseite hat, die in Bezug auf den Vorrichtungskörper (101, 111) an dem lebenden Körper angeheftet ist,
wobei der Vorrichtungskörper (101, 111) einen ersten Montageabschnitt (11d) an einer Seitenfläche hat, und
der Träger (30) einen zweiten Montageabschnitt (31c) hat, der mit dem ersten Montageabschnitt (11d) in Eingriff steht, und
wobei
der Medikamentenlösungslieferabschnitt (16, 19) und der Verbindungsanschluss (40) miteinander in einer lösbaren Weise in Fluidverbindung stehen,
der Verbindungsanschluss (40) Folgendes aufweist
einen Anschlusskörper (41), der so aufgebaut ist, dass er die Kanüle (50) hält und an dem Träger (30) zu arretieren ist,
**dadurch gekennzeichnet, dass**
der Verbindungsanschluss (40) des Weiteren Folgendes aufweist
ein Drehelement (421, 424), an dem der Medikamentenlösungslieferabschnitt (16, 19) montiert ist und das drehbar ist, wobei eine Richtung, in der ein Lumen der Kanüle (50) sich als eine Mittelachse erstreckt, in einer Richtung ist, die im Wesentlichen parallel zu dem Anordnungsflächenabschnitt (31a) ist, und
das Drehelement (421, 424) sich dreht folgend einer Drehbewegung des Vorrichtungskörpers (101, 111) in einer Montagerichtung (D1), die im Wesentlichen parallel zu dem Anordnungsflächenabschnitt (31a) ist, in einem Zustand, bei dem der Medikamentenlösungslieferabschnitt (16, 19) montiert ist.

2. Medikamentenlösungshandhabungsvorrichtung (100, 110) gemäß Anspruch 1, wobei
der Medikamentenlösungslieferabschnitt (16, 19) eine zylindrische hohle Form hat,
das Drehelement (421, 424) ein erstes Kommunikationsloch (421d) hat, das ermöglicht, dass die von dem Medikamentenlösungslieferabschnitt (16, 19) eingeleitete Medikamentenlösung dort hindurchfließt,
der Verbindungsanschluss (40) einen Pfropfen (43) aufweist, der ein zweites Kommunikationsloch (431) hat, das mit dem ersten Kommunikationsloch (421d) in Kommunikation stehen kann und an einer Außenumfangsseite des Drehelementes (421, 424) angeordnet ist, und
der Verbindungsanschluss (40) sich dreht folgend einer Drehbewegung des Vorrichtungskörpers (101, 111) in der Montagerichtung (D1), die im Wesentlichen parallel zu dem Anordnungsflächenabschnitt (31a) ist, wenn der Vorrichtungskörper (101, 111) an dem Träger (30) montiert ist, oder in einer Montagefreigaberichtung (D2), die eine Richtung ist, die zu der Montagerichtung (D1) entgegengesetzt ist, und ein Fluidverbindungszustand zwischen dem Verbindungsanschluss (40) und dem Medikamentenlösungslieferabschnitt (16, 19) in Abhängigkeit von einer Relativpositionsbeziehung zwischen dem Anschlusskörper (41) und dem Verbindungsanschluss (40) geschaltet wird.

3. Medikamentenlösungshandhabungsvorrichtung (100, 110) gemäß Anspruch 2, wobei
das Drehelement (421, 424) an dem Anschlusskörper (41) zwischen einer geschlossenen Position, an der das erste Kommunikationsloch (421d) geschlossen ist, und einer offenen Position, an der das erste Kommunikationsloch (421d) offen ist, drehbar angebracht ist, und
das Drehelement (421, 424) sich von der geschlossenen Position zu der offenen Position bewegt durch ein Drehen folgend der Drehbewegung des Vorrichtungskörpers (101, 111) in der Montagerichtung (D1) in dem Zustand, bei dem der Medikamentenlösungslieferabschnitt (16, 19) montiert ist, wodurch das erste Kommunikationsloch (421d) und das zweite Kommunikationsloch (431) miteinander in Kommunikation stehen und der Medikamentenlösungslieferabschnitt (16, 19) und der Verbindungsanschluss (40) miteinander in Fluidverbindung stehen.

4. Medikamentenlösungshandhabungsvorrichtung (100, 110) gemäß Anspruch 2 oder 3, wobei
der Medikamentenlösungslieferabschnitt (16, 19) einen ersten Eingriffsabschnitt (16b) an einem Endstückende von ihm hat,
das Drehelement (421, 424) einen zweiten Eingriffsabschnitt (421e) hat, der mit dem ersten Eingriffsabschnitt (16b) in Eingriff bringbar ist, und
der erste Eingriffsabschnitt (16b) mit dem zweiten Eingriffsabschnitt (421e) in Eingriff steht, wenn der Medikamentenlösungslieferabschnitt (16, 19) an dem Drehelement (421, 424) montiert ist, und er eine Drehkraft, die durch die Drehbewegung des Vorrichtungskörpers (101, 111) in der Montagerichtung (D1) bewirkt wird, zu dem Drehelement (421, 424) über den zweiten Eingriffsabschnitt (421e) überträgt.

5. Medikamentenlösungshandhabungsvorrichtung (100, 110) gemäß einem der Ansprüche 2 bis 4, wobei
das Drehelement (421, 424) im Inneren des Drehelementes (421, 424) einen Abdichtabschnitt (422) aufweist, der in engem Kontakt mit einer Außenumfangsfläche des montierten Medikamentenlösungslieferabschnittes (16, 19) steht.

6. Medikamentenlösungshandhabungsvorrichtung (100, 110) gemäß Anspruch 1, wobei
das Drehelement (421, 424) an einer Außenumfangsfläche des Verbindungsanschlusses (40) vorgesehen ist.

7. Medikamentenlösungshandhabungsvorrichtung (100, 110) gemäß Anspruch 6, wobei
der Medikamentenlösungslieferabschnitt (16, 19) durch ein Nadelelement (19) mit einer Nadelröhre ausgeführt ist,
der Vorrichtungskörper (101, 111) an dem Bodenflächenabschnitt (11a) eine Eingriffsvertiefung (17a) aufweist, die mit dem Medikamentenlösungslieferabschnitt (16, 19) in ihr versehen ist und die mit dem Drehelement (421, 424) in Eingriff bringbar ist, und
das Drehelement (421, 424) einen Pfropfen (43) aufweist, an dem der Medikamentenlösungslieferabschnitt (16, 19) durchdringt und montiert ist, und sich folgend der Drehbewegung des Vorrichtungskörpers (101, 111) in der Montagerichtung (D1) in einem Zustand dreht, bei dem der Medikamentenlösungslieferabschnitt (16, 19) an dem Pfropfen (43) montiert ist.

## Revendications

1. Appareil d'administration d'une solution médicamenteuse (100, 110), comprenant :
un corps de l'appareil (101, 111) qui comprend un réservoir (12) configuré pour stocker une solution médicamenteuse, un dispositif d'entraînement d'alimentation en solution médicamenteuse (23) configuré pour alimenter la solution médicamenteuse à partir du réservoir (12), un tuyau d'alimentation en solution médicamenteuse (14) à travers lequel la solution médicamenteuse alimentée à partir du réservoir (12) s'écoule, et une partie d'alimentation en solution médicamenteuse (16, 19) communiquant avec le tuyau d'alimentation en solution médicamenteuse (14) ; et
un support (30) qui comprend une partie de surface de placement (31a) sur laquelle est placée une partie de surface inférieure (11a) du corps de l'appareil (101, 111), et un port de connexion (40) disposé sur une surface supérieure de la partie de surface de placement (31a) et configuré pour maintenir une canule (50) à introduire dans un corps vivant, et qui a un côté corps vivant adhéré au corps vivant par rapport au corps de l'appareil (101, 111),
dans lequel le corps de l'appareil (101, 111) comprend une première partie de montage (11d) sur une surface latérale, et
le support (30) comprend une seconde partie de montage (31c) qui est engagée avec la première partie de montage (11d), et
dans lequel
la partie d'alimentation en solution médicamenteuse (16, 19) et le port de connexion (40) sont reliés fluidiquement l'un à l'autre de manière amovible,
le port de connexion (40) comprend
un corps du port (41) configuré pour maintenir la canule (50) et être verrouillé au support (30),
**caractérisé en ce que**
le port de connexion (40) comprend en outre
un élément rotatif (421, 424) sur lequel est montée la partie d'alimentation en solution médicamenteuse (16, 19) et qui est rotatif, avec une direction dans laquelle une lumière de la canule (50) s'étend comme axe central, dans une direction sensiblement parallèle à la partie de surface de placement (31a), et
l'élément rotatif (421, 424) tourne suite à un mouvement de rotation du corps de l'appareil (101, 111) dans une direction de montage (D1) sensiblement parallèle à la partie de surface de placement (31a) dans un état dans lequel la partie d'alimentation en solution médicamenteuse (16, 19) est montée.

2. Appareil d'administration d'une solution médicamenteuse (100, 110) selon la revendication 1, dans lequel
la partie d'alimentation en solution médicamenteuse (16, 19) a une forme cylindrique creuse,
l'élément rotatif (421, 424) a un premier trou de communication (421d) permettant à la solution médicamenteuse introduite à partir de la partie d'alimentation en solution médicamenteuse (16, 19) de s'écouler à travers celui-ci,
le port de connexion (40) comprend un bouchon (43) ayant un second trou de communication (431) pouvant communiquer avec le premier trou de communication (421d) et disposé sur un côté périphérique extérieur de l'élément rotatif (421, 424), et
le port de connexion (40) tourne suite à un mouvement de rotation du corps de l'appareil (101, 111) dans la direction de montage (D1) sensiblement parallèle à la partie de surface de placement (31a) lorsque le corps de l'appareil (101, 111) est monté sur le support (30), ou dans une direction de libération de montage (D2) qui est une direction opposée à la direction de montage (D1), et un état de connexion de fluide entre le port de connexion (40) et la partie d'alimentation en solution médicamenteuse (16, 19) est commuté en fonction d'une relation de position relative entre le corps du port (41) et le port de connexion (40).

3. Appareil d'administration d'une solution médicamenteuse (100, 110) selon la revendication 2, dans lequel
l'élément rotatif (421, 424) est attaché de manière rotative au corps du port (41) entre une position fermée dans laquelle le premier trou de communication (421d) est fermé et une position ouverte dans laquelle le premier trou de communication (421d) est ouvert, et
l'élément rotatif (421, 424) passe de la position fermée à la position ouverte en tournant suivant le mouvement de rotation du corps de l'appareil (101, 111) dans la direction de montage (D1) dans l'état dans lequel la partie d'alimentation en solution médicamenteuse (16, 19) est montée, de sorte que le premier trou de communication (421d) et le second trou de communication (431) communiquent l'un avec l'autre et la partie d'alimentation en solution médicamenteuse (16, 19) et le port de connexion (40) sont reliés fluidiquement l'un à l'autre.

4. Appareil d'administration d'une solution médicamenteuse (100, 110) selon la revendication 2 ou 3, dans lequel
la partie d'alimentation en solution médicamenteuse (16, 19) comprend une première partie d'engagement (16b) à une extrémité de pointe de celle-ci,
l'élément rotatif (421, 424) comprend une seconde partie d'engagement (421e) qui peut être engagée avec la première partie d'engagement (16b), et
la première partie d'engagement (16b) est engagée avec la seconde partie d'engagement (421e) lorsque la partie d'alimentation en solution médicamenteuse (16, 19) est montée sur l'élément rotatif (421, 424), et transmet une force de rotation causée par le mouvement de rotation du corps de l'appareil (101, 111) dans la direction de montage (D1) à l'élément rotatif (421, 424) par l'intermédiaire de la seconde partie d'engagement (421e).

5. Appareil d'administration d'une solution médicamenteuse (100, 110) selon l'une quelconque des revendications 2 à 4, dans lequel
l'élément rotatif (421, 424) comprend, à l'intérieur de l'élément rotatif (421, 424), une partie d'étanchéité (422) qui est en contact étroit avec une surface périphérique extérieure de la partie d'alimentation en solution médicamenteuse (16, 19) montée.

6. Appareil d'administration d'une solution médicamenteuse (100, 110) selon la revendication 1, dans lequel
l'élément rotatif (421, 424) est disposé sur une surface périphérique extérieure du port de connexion (40).

7. Appareil d'administration d'une solution médicamenteuse (100, 110) selon la revendication 6, dans lequel
la partie d'alimentation en solution médicamenteuse (16, 19) est réalisée par un élément d'aiguille (19) avec un tube d'aiguille,
le corps de l'appareil (101, 111) comprend, au niveau de la partie de surface inférieure (11a), une évidement engageante (17a) qui est pourvu de la partie d'alimentation en solution médicamenteuse (16, 19) à l'intérieur et qui peut être engagé avec l'élément rotatif (421, 424), et
l'élément rotatif (421, 424) comprend un bouchon (43) dans lequel la partie d'alimentation en solution médicamenteuse (16, 19) pénètre et est montée, et tourne en suivant le mouvement de rotation du corps de l'appareil (101, 111) dans la direction de montage (D1) dans un état dans lequel la partie d'alimentation en solution médicamenteuse (16, 19) est montée sur le bouchon (43).
